# EUROPEAN PATENT SPECIFICATION

(11) **EP 3 828 289 B1**
(45) Date of publication and mention of the grant of the patent: **14.10.2026**
(21) Application number: 18927433.5
(22) Date of filing: 26.07.2018
(51) Int. Cl.: C12Q 1/6886, C12Q 1/6883

(54) **METHOD AND KIT FOR IDENTIFYING GASTRIC CANCER STATUS**
VERFAHREN UND KIT ZUR IDENTIFIZIERUNG EINES MAGENKREBSZUSTANDS
MÉTHODE ET KIT POUR IDENTIFIER L'ÉTAT D'UN CANCER GASTRIQUE

(43) Date of publication of application: 02.06.2021
(73) Proprietor: Excellen Medical Technology Co., Ltd., Science and Technology Park, Changpin District Beijing 102200 (CN)
(72) Inventor: LI, Mingming, Beijing 102200 (CN); LI, Shuyu, Beijing 102200 (CN); CHEN, Yanli, Beijing 102200 (CN); XU, Chunye, Beijing 102200 (CN); PU, Jue, Beijing 102200 (CN)
(74) Representative: Glück Kritzenberger Patentanwälte PartGmbB
(86) International application number: PCT/CN2018/097297
(87) International publication number: WO 2020/019269

(56) References cited:
- EP-A2- 3 301 194
- WO-A1-2014/073785
- WO-A1-2016/019900
- WO-A2-2009/034481
- CN-A- 104 745 681
- CN-A- 104 812 899
- CN-A- 107 904 313
- US-A1- 2018 016 639
- WANG NA ET AL: "Site-specific Hypermethylation ofRUNX3Predicts Poor Prognosis in Gastric Cancer", ARCHIVES OF MEDICAL RESEARCH, INSTITUTO MEXICANO DEL SEGURO SOCIAL, MEXICO, MX, vol. 47, no. 4, 22 September 2016 (2016-09-22), pages 285 - 292, XP029738718, ISSN: 0188-4409, DOI: 10.1016/J.ARCMED.2016.07.011
- YAMADA HIDETAKA ET AL: "Absence of germline mono-allelic promoter hypermethylation of the CDH1 gene in gastric cancer patients", MOLECULAR CANCER, BIOMED CENTRAL, LONDON, GB, vol. 8, no. 1, 12 August 2009 (2009-08-12), pages 63, XP021058751, ISSN: 1476-4598, DOI: 10.1186/1476-4598-8-63
- LUO JUN ET AL: "[Methylation status of CDH1 gene in preoperative abdominal lavage of patients with gastric cancer and its clinical significance].", ZHONGHUA WEI CHANG WAI KE ZA ZHI = CHINESE JOURNAL OF GASTROINTESTINAL SURGERYCHINA JUL 2012, vol. 15, no. 07, 15 July 2012 (2012-07-15), pages 710 - 714, XP009532956
- LUO JUN ET AL: "[Methylation status of CDH1 gene in preoperative abdominal lavage of patients with gastric cancer and its clinical significance].", ZHONGHUA WEI CHANG WAI KE ZA ZHI = CHINESE JOURNAL OF GASTROINTESTINAL SURGERY JUL 2012, vol. 15, no. 7, July 2012 (2012-07-01), pages 710 - 714, XP009532956, ISSN: 1671-0274
- CUI HE ET AL: "Deregulation between miR-29b/c and DNMT3A Is Associated with Epigenetic Silencing of the CDH1 Gene, Affecting Cell Migration and Invasion in Gastric Cancer", PLOS ONE, vol. 10, no. 4, 15 April 2015 (2015-04-15), pages e0123926, XP055883541, DOI: 10.1371/journal.pone.0123926
- DATABASE Geneseq [online] 9 October 2014 (2014-10-09), "Human CDH1 gene methylation status detecting forward primer.", XP002805446, retrieved from EBI accession no. GSN:BBM25316 Database accession no. BBM25316
- DATABASE Geneseq [online] 28 October 2010 (2010-10-28), "Human BAX gene forward methylation-specific PCR primer, SEQ ID NO: 5.", XP002805447, retrieved from EBI accession no. GSN:AYI28209 Database accession no. AYI28209
- DATABASE Geneseq [online] 1 January 2015 (2015-01-01), "Human CDH1 gene promoter antisense qMSP primer, SEQ 258.", XP002805448, retrieved from EBI accession no. GSN:BBQ10760 Database accession no. BBQ10760
- DATABASE Geneseq [online] 17 April 2008 (2008-04-17), "Human CDH1 methylation-specific forward PCR primer SEQ ID 9.", XP002805449, retrieved from EBI accession no. GSN:AQX95592 Database accession no. AQX95592
- DATABASE Geneseq [online] 9 September 2004 (2004-09-09), "PCR primer amplifies promoter CpG islands of cancer related genes Seq183.", XP002805450, retrieved from EBI accession no. GSN:ADQ77501 Database accession no. ADQ77501
- MACHADO J C ET AL: "E-cadherin gene (CDH1) promoter methylation as the second hit in sporadic diffuse gastric carcinoma", ONCOGENE, NATURE PUBLISHING GROUP UK, LONDON, vol. 20, no. 12, 22 March 2001 (2001-03-22), pages 1525 - 1528, XP037735040, ISSN: 0950-9232, DOI: 10.1038/SJ.ONC.1204234
- ANNE M SLAVOTINEK: "Methylation of the CDH1 promoter as the second genetic hit in hereditary diffuse gastric cancer", 1 September 2000 (2000-09-01), pages 16 - 17, XP93168811, Retrieved from the Internet <URL:https://www.nature.com/articles/ng0900_16>
- QI-MING YU: "CDH1 methylation in preoperative peritoneal washes is an independent prognostic factor for gastric cancer", JOURNAL OF SURGICAL ONCOLOGY, vol. 106, no. 6, 18 April 2012 (2012-04-18), US, pages 765 - 771, XP93168816, ISSN: 0022-4790, Retrieved from the Internet <URL:https://onlinelibrary.wiley.com/doi/full-xml/10.1002/jso.23116> DOI: 10.1002/jso.23116
- DEMAO YAO ET AL: "Quantitative assessment of gene methylation and their impact on clinical outcome in gastric cancer", CLINICA CHIMICA ACTA, ELSEVIER BV, AMSTERDAM, NL, vol. 413, no. 7, 15 January 2012 (2012-01-15), pages 787 - 794, XP028463582, ISSN: 0009-8981, [retrieved on 20120121], DOI: 10.1016/J.CCA.2012.01.013
- CAO CHANG-QI ET AL: "Circulating methylated Septin 9 and ring finger protein 180 for noninvasive diagnosis of early gastric cancer", TRANSLATIONAL CANCER RESEARCH, vol. 9, no. 11, 1 November 2020 (2020-11-01), pages 7012 - 7021, XP093364329, ISSN: 2218-676X, DOI: 10.21037/tcr-20-1330
- ZENG YAO , LI XIAO-XING , YU JUN : "Progress of the Methylation Study and Related Prognostic Biomarker in Gastric Cancer", NEW MEDICINE, vol. 45, no. 2, 15 February 2014 (2014-02-15), pages 71 - 78, XP055778706, ISSN: 0253-9802, DOI: 10.3969/g.issn.0253-9802.2014.02.001
- QU YIPING; DANG SIWEN; HOU PENG: "Gene Methylation in Gastric Cancer", CLINICA CHIMICA ACTA, vol. 424, 10 May 2013 (2013-05-10), pages 53 - 65, XP028708538, ISSN: 0009-8981, DOI: 10.1016/j.cca.2013.05.002

## Description

### FIELD OF THE INVENTION

The present invention relates to a method and a kit for identifying a gastric cancer status in a subject.

### BACKGROUND

Gastric cancer is one of the most common malignant tumors in the world. According to the data provided by WHO: there are more than 1.5 million gastric cancer patients worldwide, and its mortality rate ranks third among global cancer deaths. Gastric cancer most commonly occurs in East Asia and Eastern Europe. China accounts for more than 40% of new gastric cancer cases and related deaths worldwide, ranking among the top in the world. Together with Japan and South Korea, the gastric cancer rate accounts for about 2/3 of the global patients. The International Agency for Research on Cancer (IARC), a subsidiary of WHO, speculates that the high incidence of gastric cancer in the three Northeast Asian countries may be related to race (genetic), dietary habits, and aging. As far as the incidence of gastric cancer is concerned, the incidence of men are usually 2-3 times that of women, and it will increase rapidly after the age of 35. The highest median age of onset of gastric cancer is 50-60 years old and tends to be younger. The disease stage of gastric cancer is the most important prognostic factor. The 5-year survival rate of patients with early gastric cancer can reach more than 90%, while the advanced stage is less than 10%. However, when gastric cancer patients are treated in medical institutions, the disease has often progressed to the middle and late stages, and its treatment effect and prognosis are poor. For example, in China, the rate of early gastric cancer patients is only 22.1%, the rate of stage III patients is 38.5%, and the rate of stage IV patients is 15.4%, which directly leads to the poor prognosis of Chinese gastric cancer patients. The 5-year relative survival rate of gastric cancer in most countries and regions in the world is only about 20%. The 5-year survival rate of stage I and II gastric cancer in Japan is more than 70%, which is related to the population gastric cancer screening program in Japan since the 1960s, and a relatively high proportion of early cases of gastric cancer.

Gastric cancer is mainly diagnosed by histological specimens taken out by endoscopy, but diagnostic techniques have not greatly reduced the mortality of gastric cancer patients. Therefore, improvement of the survival rate of gastric cancer patients depends on early diagnosis, and screening and mining valuable biological markers of early gastric cancer has become an urgent problem to be solved. Because imaging techniques failed to show good results in the early screening of gastric cancer, people began to turn their attention to molecular markers for early diagnosis of gastric cancer. Unfortunately, no molecular marker with high sensitivity and specificity has been found so far. Compared with imaging examination, endoscopy can directly observe the shape of the lesion, and the biopsy has a high accuracy rate. The positive coincidence rate of CT simulated gastroscopy in the diagnosis of early gastric cancer is more than 70%, and the smallest diameter that can show mucosal lesions is about 1 cm. However, the process of gastroscopy is very painful, and less than 10% of patients can really accept regular endoscopy.

In recent years, research on gastric cancer epigenetics has made rapid progress, especially in DNA methylation. It has been found that many specific tumor-related genes have different degrees of methylation status change in the early stage of gastric cancer, which provides a new opportunity for the exploration of markers for early diagnosis of gastric cancer.

Abnormal DNA methylation of the genome and the occurrence of tumors have always been one of the hotspots in medical research. Cell cycle, DNA repair, angiogenesis, apoptosis, etc. all involve the methylation of related genes. The most likely regulatory role of DNA hypermethylation is to suppress the expression of key genes, thereby determining the fate of a cell. For example, the study of abnormal DNA methylation in tumor cells has made many significant advances in various tumors. In mammals, methylation only affects a cytosine in front of a guanine (CpG) on a DNA strand. The methylation distribution of CpG dinucleotides in normal cells is not uniform. About 50% of genes have CpG islands with concentrated distribution of CpGs in the promoter region, with lengths ranging from 0.5 to 2kb. This region is closely related to gene transcription regulation. In humans, the methylation of CpG islands in certain gene regulatory regions occurs frequently in relevant cancer cell tissues, showing a correlation with the onset, disease progression, prognosis, drug sensitivity, ect. of certain tumors. To date, gene methylation abnormalities have been found in most human tumors. Studies have found that epigenetic coding in cancer cells is disturbed, first of all manifested in the disturbance of DNA methylation level, also known as methylation rearrangement. Since the local hypermethylation of a CpG island in a tumor suppressor gene is earlier than the malignant proliferation of cells, the detection of DNA methylation can be used for the early diagnosis of tumorigenesis. Methylation of cancer-related genes is also an early event of gastric cancer, so the methylation status of related genes has become an effective indicator for the risk prediction of early gastric cancer. Even so, there is still a lack of means to effectively detect the methylation status of these cancer-related genes and process the detected results.

At present, what is urgently needed in the field of gastrointestintal oncology is a clinical test that mini-invasively evaluates and predicts the presence of a gastric cancer.

### SUMMARY

In order to solve the above problems, in one aspect, a method for identifying a gastric cancer status in a subject is provided herein, which comprises the following steps: 1) collecting a biological sample from the subject; 2) detecting methylation level(s) of a biomarker gene in the biological sample, wherein the biomarker genes comprise CDH1, DAPK, RASSF1A, RNF180 and Septin9; and 3) comparing the methylation levels detected in step 2) with normal methylation levels of the corresponding biomarker genes in a population to determine the gastric cancer status in the subject, wherein the detection of the methylation level of the CDH1 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the CDH1 gene or a fragment thereof which is bisulfite-treated in the biological sample as a template; the detection of the methylation level of the DAPK gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the DAPK gene or a fragment thereof which is bisulfite-treated in the biological sample as a template; the detection of the methylation level of the RASSF1A gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the RASSF1A gene or a fragment thereof which is bisulfite-treated in the biological sample as a template; the detection of the methylation level of the RNF180 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the RNF180 gene or a fragment thereof which is bisulfite-treated in the biological sample as a template; the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the Septin9 gene or a fragment thereof which is bisulfite-treated in the biological sample as a template.

In some embodiments, the step 2) comprises extracting DNA from the biological sample and treating the extracted DNA with a bisulfite, so that unmethylated cytosine residues in the DNA are deaminated, and methylated cytosine residues remain unchanged.

In some preferred embodiments, the biomarker genes are CDH1, DAPK, PAX5, RASSF1A, Reprimo, RNF180, RUNX3, SDC2, Septin9 and TCF4.

In some embodiments, step 2) comprises detecting the methylation levels of a target region within the biomarker genes, and wherein the target region is a nucleotide sequence of at least 15 bases in the biomarker genes, or a complementary sequence thereof.

In some preferred embodiments, in step 2) the detection of the methylation level of the CDH1 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the CDH1 gene or a fragment thereof which is bisulfite-treated in the biological sample as a template;
the detection of the methylation level of the DAPK gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the DAPK gene or a fragment thereof which is bisulfite-treated in the biological sample as a template;
the detection of the methylation level of the PAX5 gene comprises the use of a primer pair having the sequences as set forth in SEQ ID NOs: 27 and 28 or a primer pair having the sequences as set forth in SEQ ID NOs: 31 and 32 to carry out a PCR amplification reaction, with the PAX5 gene or a fragment thereof which is bisulfite-treated in the biological sample as a template;
the detection of the methylation level of the RASSF1A gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the RASSF1A gene or a fragment thereof which is bisulfite-treated in the biological sample as a template;
the detection of the methylation level of the Reprimo gene comprises the use of a primer pair having the sequences as set forth in SEQ ID NOs: 43 and 44 or a primer pair having the sequences as set forth in SEQ ID NOs: 47 and 48 to carry out a PCR amplification reaction, with the Reprimo gene or a fragment thereof which is bisulfite-treated in the biological sample as a template;
the detection of the methylation level of the RNF180 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the RNF180 gene or a fragment thereof which is bisulfite-treated in the biological sample as a template;
the detection of the methylation level of the RUNX3 gene comprises the use of a primer pair having the sequences as set forth in SEQ ID NOs: 63 and 64, a primer pair having the sequences as set forth in SEQ ID NOs: 67 and 68 or a primer pair having the sequences as set forth in SEQ ID NOs: 71 and 72 to carry out a PCR amplification reaction, with the RUNX3 gene or a fragment thereof which is bisulfite-treated in the biological sample as a template;
the detection of the methylation level of the SDC2 gene comprises the use of a primer pair having the sequences as set forth in SEQ ID NOs: 75 and 76, a primer pair having the sequences as set forth in SEQ ID NOs: 79 and 80 or a primer pair having the sequences as set forth in SEQ ID NOs: 83 and 84 to carry out a PCR amplification reaction, with the SDC2 gene or a fragment thereof which is bisulfite-treated in the biological sample as a template;
the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the Septin9 gene or a fragment thereof which is bisulfite-treated in the biological sample as a template; and
the detection of the methylation level of the TCF4 gene comprises the use of a primer pair having the sequences as set forth in SEQ ID NOs: 95 and 96 or a primer pair having the sequences as set forth in SEQ ID NOs: 99 and 100 to carry out a PCR amplification reaction, with the TCF4 gene or a fragment thereof which is bisulfite-treated in the biological sample as a template.

In some preferred embodiments, in step 2) the detection of the methylation level of the CDH1 gene comprises the use of a primer pair having the sequences as set forth in SEQ ID NOs: 11 and 12 and a blocking primer having the sequence as set forth in SEQ ID NO: 13, or a primer pair having the sequences as set forth in SEQ ID NOs: 15 and 16 and a blocking primer having the sequence as set forth in SEQ ID NO: 17 to carry out a PCR amplification reaction, with the bisulfite-treated CDH1 gene or a fragment thereof in the biological sample as a template; the detection of the methylation level of the DAPK gene comprises the use of a primer pair having the sequences as set forth in SEQ ID NOs: 19 and 20 and a blocking primer having the sequence as set forth in SEQ ID NO: 21, or a primer pair having the sequences as set forth in SEQ ID NOs: 23 and 24 and a blocking primer having the sequence as set forth in SEQ ID NO: 25 to carry out a PCR amplification reaction, with the bisulfite-treated DAPK gene or a fragment thereof in the biological sample as a template; the detection of the methylation level of the PAX5 gene comprises the use of a primer pair having the sequences as set forth in SEQ ID NOs: 27 and 28 and a blocking primer having the sequence as set forth in SEQ ID NO: 29, or a primer pair having the sequences as set forth in SEQ ID NOs: 31 and 32 and a blocking primer having the sequence as set forth in SEQ ID NO: 33 to carry out a PCR amplification reaction, with the bisulfite-treated PAX5 gene or a fragment thereof in the biological sample as a template; the detection of the methylation level of the RASSF1A gene comprises the use of a primer pair having the sequences as set forth in SEQ ID NOs: 35 and 36 and a blocking primer having the sequence as set forth in SEQ ID NO: 37, or a primer pair having the sequences as set forth in SEQ ID NOs: 39 and 40 and a blocking primer having the sequence as set forth in SEQ ID NO: 41 to carry out a PCR amplification reaction, with the bisulfite-treated RASSF1A gene or a fragment thereof in the biological sample as a template; the detection of the methylation level of the Reprimo gene comprises the use of a primer pair having the sequences as set forth in SEQ ID NOs: 43 and 44 and a blocking primer having the sequence as set forth in SEQ ID NO: 45, or a primer pair having the sequences as set forth in SEQ ID NOs: 47 and 48 and a blocking primer having the sequence as set forth in SEQ ID NO: 49 to carry out a PCR amplification reaction, with the bisulfite-treated Reprimo gene or a fragment thereof in the biological sample as a template;the detection of the methylation level of the RNF180 gene comprises the use of a primer pair having the sequences as set forth in SEQ ID NOs: 51 and 52 and a blocking primer having the sequence as set forth in SEQ ID NO: 53, a primer pair having the sequences as set forth in SEQ ID NOs: 55 and 56 and a blocking primer having the sequence as set forth in SEQ ID NO: 57,or a primer pair having the sequences as set forth in SEQ ID NOs: 59 and 60 and a blocking primer having the sequence as set forth in SEQ ID NO: 61 to carry out a PCR amplification reaction, with the bisulfite-treated RNF180 gene or a fragment thereof in the biological sample as a template; the detection of the methylation level of the RUNX3 gene comprises the use of a primer pair having the sequences as set forth in SEQ ID NOs: 63 and 64 and a blocking primer having the sequence as set forth in SEQ ID NO: 65, a primer pair having the sequences as set forth in SEQ ID NOs: 67 and 68 and a blocking primer having the sequence as set forth in SEQ ID NO: 69, or a primer pair having the sequences as set forth in SEQ ID NOs: 71 and 72 and a blocking primer having the sequence as set forth in SEQ ID NO: 73 to carry out a PCR amplification reaction, with the bisulfite-treated RUNX3 gene or a fragment thereof in the biological sample as a template; the detection of the methylation level of the SDC2 gene comprises the use of a primer pair having the sequences as set forth in SEQ ID NOs: 75 and 76 and a blocking primer having the sequence as set forth in SEQ ID NO: 77, a primer pair having the sequences as set forth in SEQ ID NOs: 79 and 80 and a blocking primer having the sequence as set forth in SEQ ID NO: 81, or a primer pair having the sequences as set forth in SEQ ID NOs: 83 and 84 and a blocking primer having the sequence as set forth in SEQ ID NO: 85 to carry out a PCR amplification reaction, with the bisulfite-treated SDC2 gene or a fragment thereof in the biological sample as a template; the detection of the methylation level of the Septin9 gene comprises the use of a primer pair having the sequences as set forth in SEQ ID NOs: 87 and 88 and a blocking primer having the sequence as set forth in SEQ ID NO: 89, or a primer pair having the sequences as set forth in SEQ ID NOs: 91 and 92 and a blocking primer having the sequence as set forth in SEQ ID NO: 93 to carry out a PCR amplification reaction, with the bisulfite-treated Septin9 gene or a fragment thereof in the biological sample as a template; and the detection of the methylation level of the TCF4 gene comprises the use of a primer pair having the sequences as set forth in SEQ ID NOs: 95 and 96 and a blocking primer having the sequence as set forth in SEQ ID NO: 97, or a primer pair having the sequences as set forth in SEQ ID NOs: 99 and 100 and a blocking primer having the sequence as set forth in SEQ ID NO: 101 to carry out a PCR amplification reaction, with the bisulfite-treated TCF4 gene or a fragment thereof in the biological sample as a template, wherein the blocking primers have a 3' end modification which prevents the extension and amplification of a DNA polymerase.

In some embodiments, step 3) comprises determining the gastric cancer status in the subject according to the methylation levels of the biomarker genes based on a logistic regression.

In another aspect, a kit for use in a method of identifying a gastric cancer status in a subject is provided herein, which comprises a primer pair for detecting methylation levels of a biomarker gene in a biological sample from the subject, wherein the primer pair is used to carry out a PCR amplification reaction with the biomarker gene or a fragment thereof which is bisulfite-treated as a template; and the biomarker genes comprise CDH1, DAPK, RASSF1A, RNF180 and Septin9, wherein the primer pair used for the detection of the methylation level of CDH1 has the sequences as set forth in SEQ ID NOs: 11 and 12 or the sequences as set forth in SEQ ID NOs: 15 and 16; the primer pair used for the the detection of the methylation level of DAPK has the sequences as set forth in SEQ ID NOs: 19 and 20 or the sequences as set forth in SEQ ID NOs: 23 and 24; the primer pair used for the the detection of the methylation level of RASSF1A has the sequences as set forth in SEQ ID NOs: 35 and 36 or the sequences as set forth in SEQ ID NOs: 39 and 40; the primer pair used for the the detection of the methylation level of RNF180 has the sequences as set forth in SEQ ID NOs: 51 and 52, the sequences as set forth in SEQ ID NOs: 55 and 56 or the sequences as set forth in SEQ ID NOs: 59 and 60; and the primer pair used for the the detection of the methylation level of Septin9 has the sequences as set forth in SEQ ID NOs: 87 and 88 or the sequences as set forth in SEQ ID NOs: 91 and 92.

In some preferred embodiments, the biomarker genes of the kit for use in a method of identifying a gastric cancer status CDH1, DAPK, PAX5, RASSF1A, Reprimo, RNF180, RUNX3, SDC2, Septin9 and TCF4.

In some preferred embodiments, in the kit for use in a method of identifying a gastric cancer status
the primer pair used for the the detection of the methylation level of CDH1 has the sequences as set forth in SEQ ID NOs: 11 and 12 or the sequences as set forth in SEQ ID NOs: 15 and 16;
the primer pair used for the the detection of the methylation level of DAPK has the sequences as set forth in SEQ ID NOs: 19 and 20 or the sequences as set forth in SEQ ID NOs: 23 and 24;
the primer pair used for the the detection of the methylation level of PAX5 has the sequences as set forth in SEQ ID NOs: 27 and 28 or the sequences as set forth in SEQ ID NOs: 31 and 32;
the primer pair used for the the detection of the methylation level of RASSF1A has the sequences as set forth in SEQ ID NOs: 35 and 36 or the sequences as set forth in SEQ ID NOs: 39 and 40;
the primer pair used for the the detection of the methylation level of Reprimo has the sequences as set forth in SEQ ID NOs: 43 and 44 or has the sequences as set forth in SEQ ID NOs: 47 and 48;
the primer pair used for the the detection of the methylation level of RNF180 has the sequences as set forth in SEQ ID NOs: 51 and 52, the sequences as set forth in SEQ ID NOs: 55 and 56 or the sequences as set forth in SEQ ID NOs: 59 and 60;
the primer pair used for the the detection of the methylation level of RUNX3 has the sequences as set forth in SEQ ID NOs: 63 and 64, the sequences as set forth in SEQ ID NOs: 67 and 68, or the sequences as set forth in SEQ ID NOs: 71 and 72;
the primer pair used for the the detection of the methylation level of SDC2 has the sequences as set forth in SEQ ID NOs: 75 and 76, the sequences as set forth in SEQ ID NOs: 79 and 80 or the sequences as set forth in SEQ ID NOs: 83 and 84;
the primer pair used for the the detection of the methylation level of Septin9 has the sequences as set forth in SEQ ID NOs: 87 and 88 or the sequences as set forth in SEQ ID NOs: 91 and 92; and
the primer pair used for the the detection of the methylation level of TCF4 has the sequences as set forth in SEQ ID NOs: 95 and 96 or the sequences as set forth in SEQ ID NOs: 99 and 100.

In preferred enbodiments, the kit for use in a method of identifying a gastric cancer status may further comprise a blocking primer, wherein the blocking primer used in combination with the primer pair having the sequences as set forth in SEQ ID NO: 11 and 12 has the sequence as set forth in SEQ ID NO: 13; the blocking primer used in combination with the primer pair having the sequences as set forth in SEQ ID NO: 15 and 16 has the sequence as set forth in SEQ ID NO: 17; the blocking primer used in combination with the primer pair having the sequences as set forth in SEQ ID NO: 19 and 20 has the sequence as set forth in SEQ ID NO: 21; the blocking primer used in combination with the primer pair having the sequences as set forth in SEQ ID NO: 23 and 24 has the sequence as set forth in SEQ ID NO: 25; the blocking primer used in combination with the primer pair having the sequences as set forth in SEQ ID NO: 27 and 28 has the sequence as set forth in SEQ ID NO: 29; the blocking primer used in combination with the primer pair having the sequences as set forth in SEQ ID NO: 31 and 32 has the sequence as set forth in SEQ ID NO: 33; the blocking primer used in combination with the primer pair having the sequences as set forth in SEQ ID NO: 35 and 36 has the sequence as set forth in SEQ ID NO: 37; the blocking primer used in combination with the primer pair having the sequences as set forth in SEQ ID NO: 39 and 40 has the sequence as set forth in SEQ ID NO: 41; the blocking primer used in combination with the primer pair having the sequences as set forth in SEQ ID NO: 43 and 44 has the sequence as set forth in SEQ ID NO: 45; the blocking primer used in combination with the primer pair having the sequences as set forth in SEQ ID NO: 47 and 48 has the sequence as set forth in SEQ ID NO: 49; the blocking primer used in combination with the primer pair having the sequences as set forth in SEQ ID NO: 51 and 52 has the sequence as set forth in SEQ ID NO: 53; the blocking primer used in combination with the primer pair having the sequences as set forth in SEQ ID NO: 55 and 56 has the sequence as set forth in SEQ ID NO: 57; the blocking primer used in combination with the primer pair having the sequences as set forth in SEQ ID NO: 59 and 60 has the sequence as set forth in SEQ ID NO: 61; the blocking primer used in combination with the primer pair having the sequences as set forth in SEQ ID NO: 63 and 64 has the sequence as set forth in SEQ ID NO: 65; the blocking primer used in combination with the primer pair having the sequences as set forth in SEQ ID NO: 67 and 68 has the sequence as set forth in SEQ ID NO: 69; the blocking primer used in combination with the primer pair having the sequences as set forth in SEQ ID NO: 71 and 72 has the sequence as set forth in SEQ ID NO: 73; the blocking primer used in combination with the primer pair having the sequences as set forth in SEQ ID NO: 75 and 76 has the sequence as set forth in SEQ ID NO: 77; the blocking primer used in combination with the primer pair having the sequences as set forth in SEQ ID NO: 79 and 80 has the sequence as set forth in SEQ ID NO: 81; the blocking primer used in combination with the primer pair having the sequences as set forth in SEQ ID NO: 83 and 84 has the sequence as set forth in SEQ ID NO: 85; the blocking primer used in combination with the primer pair having the sequences as set forth in SEQ ID NO: 87 and 88 has the sequence as set forth in SEQ ID NO: 89; the blocking primer used in combination with the primer pair having the sequences as set forth in SEQ ID NO: 91 and 92 has the sequence as set forth in SEQ ID NO: 93; the blocking primer used in combination with the primer pair having the sequences as set forth in SEQ ID NO: 95 and 96 has the sequence as set forth in SEQ ID NO: 97; and the blocking primer used in combination with the primer pair having the sequences as set forth in SEQ ID NO: 99 and 100 has the sequence as set forth in SEQ ID NO: 101, wherein the blocking primers have a 3' end modification which prevents the extension and amplification of a DNA polymerase.

In preferred enbodiments, the kit for use in a method of identifying a gastric cancer status further comprises a DNA extraction reagent and a bisulfite reagent. Preferably, the bisulfite reagent comprises sodium bisulfite.

In preferred embodiments, the kit further comprises an instruction that discribes how to use the kit and process detection results with a logistic regression.

### BRIEF DESCRIPTION OF THE DRAWINGS

Figure 1 shows the receiver operating characteristic (ROC) curves of the methylation levels of 10 biomarker genes.
Figure 2 shows the methylation level distribution of 10 biomarker genes in different gastric cancer stages (indicated by Ct values). Figure 2A shows the methylation level distribution of CDH1, DAPK, PAX5, RASSF1A, Reprimo and RNF180, and Figure 2B shows the methylation level distribution of RUNX3, SDC2, Septin9 and TCF4.
Figure 3 shows the methylation level distribution of 10 biomarker genes in different gastric cancer subtypes (indicated by Ct values). Figure 3A shows the methylation level distribution of CDH1, DAPK, PAX5, RASSF1A, Reprimo and RNF180, and Figure 2B shows the methylation level distribution of RUNX3, SDC2, Septin9 and TCF4.
Figure 4 shows the receiver operating characteristic (ROC) curve of a logistic regression model constructed with 10 marker genes;
Figure 5 shows the receiver operating characteristic (ROC) curve of a logistic regression model constructed with the 5 most characteristic marker genes.

### DETAILED DESCRIPTION

Unless otherwise stated, the technical terms used in this application have the meanings generally understood by those skilled in the art to which the present invention belongs.

The present application in one aspect relates to a method for identifying a gastric cancer status in a subject, which comprises the following steps: 1) collecting a biological sample from the subject; 2) detecting the methylation level(s) of a biomarker gene in the biological sample, wherein the biomarker gene(s) is/are selected from one or more of the following genes: CDH1 (E-cardherin), DAPK (Death-associated protein kinase-1), PAX5 (Paired box 5), RASSF1A (Ras-association domain family 1 isoform A), Reprimo, RNF180 (Ring finger protein180), RUNX3 (Runt-related transcription factor3), SDC2 (Syndecan 2), Septin9 and TCF4 (Transcription factor 4); and 3) comparing the methylation levels detected in step 2) with the normal methylation levels of the corresponding biomarker gene(s) in a population to determine the gastric cancer status in the subject.

The term "subject" as used herein refers to an individual (preferably a human) suffering from or suspected of having a certain disease, or, when predicting the susceptibility, "subject" may also include healthy individuals. The term is generally used interchangeably with "patient", "test subject", "treatment subject", and the like.

The term "population" as used herein generally refers to healthy people. When referring to a specific disease (such as a gastric cancer), a "population" may include individuals who do not suffer from such specific disease but may suffer from other diseases. In addition, it is also possible to select only some individuals as the "population" based on characteristics such as, age, gender, health status, smoking or not, etc. A "normal methylation level in a population" can be obtained by detecting enough individuals or can be found in an existing clinical literature. In some cases, this normal level refers to no methylation.

The term "gastric cancer status" used herein includes a gastric cancer susceptibility and the presence, progression, subtype, and/or stage of a gastric cancer. In some embodiments, the subject's susceptibility to a gastric cancer can be predicted based on the methylation levels of the biomarker gene(s) in the subject. In other embodiments, the subject may be identified for the presence of a gastric cancer based on the methylation levels of the biomarker gene(s) in the subject; and if a gastric cancer is present, the subtype and/or the stage of the gastric cancer may be identified. Gastric cancer subtypes may include adenocarcinoma, mucoid carcinoma, undifferentiated carcinoma, and other gastric cancers. The gastric cancer stages may include stage I (IA, IB, or IC), stage II, stage III, and stage IV. In some embodiments, the gastric cancer is a stage I gastric cancer. In some embodiments, the gastric cancer is a stage II gastric cancer. In some embodiments, the gastric cancer is a stage III gastric cancer. In other embodiments, the gastric cancer is a stage IV gastric cancer.

In the method of the present invention, treatment of the subject, for example, including performing more tests on the subject, performing a surgery, giving medications, and taking no further actions, may also be arranged based on the stage of the gastric cancer. In other embodiments, the method of the present invention further comprises measuring the methylation levels of one or more biomarker genes of CDH1, DAPK, PAX5, RASSF1A, Reprimo, RNF180, RUNX3, SDC2, Septin9 and TCF4 genes or fragments thereof in the subject after the subject is treated, and correlating the measurement results with the gastric cancer status to identify whether the treatment results in a change in the gastric cancer status in the subject. In some embodiments, the correlation is performed by a classification algorithm of a software.

The detection of the methylation levels in step 2) comprises extracting DNA from a biological sample, treating it with a bisulfite, and then carrying out a PCR amplification reaction by using a methylation-specific primer pair. The bisulfite treatment causes unmethylated cytosine residues in a double-stranded DNA molecule to deaminate to be uracils; while methylated cytosine residues remain unchanged. As a result, in the subsequent PCR amplification reaction, methylated cytosine residue sites on a template are paired with guanine residues in a primer as cytosine residues, while unmethylated cytosine residue sites are paired with adenine residues in a primer as uracil residues. The inventors designed multiple primer pairs for each biomarker gene to detect the methylation level of a target region within each biomarker gene. The target regions are selected from the fragments of at least 15 consecutive bases in the sequences as set forth in SEQ ID NOs: 1-10 (corresponding to CDH1, DAPK, PAX5, RASSF1A, Reprimo, RNF180, RUNX3, SDC2, Septin9 and TCF4 gene), respectively; and the nucleic acid sequences of the primer pairs are respectively identical, complementary or hybridizable to the above target regions. The primer pairs provided herein make use of the methylation difference to detect the methylation levels of the target regions within the biomarker genes. When a target region of a biomarker gene is not methylated, the primer pair used cannot effectively pair with and bind to the target region (treated with bisulfite) which is used as a template in the PCR amplification reaction, and cannot (or rarely) generate amplification products; and when the target gene of the biomarker gene is methylated, the primer pair used is able to effectively pair with and bind to the target region (treated with bisulfite) which is used as a template in the PCR amplification reaction, and thus generate amplification products. The differences of these amplification reactions can be monitored in real time during the amplification reactions, or can be judged by detecting the amplification products. After many experiments, the inventors screened out multiple primer pairs for the biomarker genes (see below), which can be used alone or in combination to help identify the gastric cancer status in the subject.

| |
|---|
| SEQ ID NO: 1 DNA sequence of human CDH1 |
| aaaagaactcagccaagtgtaaaagccctttctgatcccaggtcttagtgagccaccggcggggctgggattcgaacccagtggaatcagaaccgtgc aggtcccataacccacctagaccctagcaactccaggctagagggtcaccgcgtctatgcgaggccgggtgggcggccgtcagctccgccctgggga ggggtccgcgctgctgattggctgtggccggcaggtgaaccctcagccaatcagcggtacgggggcggtgcctccggggctcacctggctgcagcca cgcacccccctctcagtggcgtcggaactgcaaagcacctgtgagcttgcggaagtcagttcagactccagcccgctccagcccggcccgacccgaccgc acccggcgcctgccctcgctcggcgtccccggccagccatgggcccttggagccgcagcctctcggcgctgctgctgctgctgcaggtaccccggatccc ctgacttgcgagggacgcattcggccgcaagctccgcgccccagccctgcgccccttcctctcccgtcgtcaccgcttcccttcttccaagaaagttcggg tcctgaggagcggagcggcctggaagcctcgcgcgctccggac |

| |
|---|
| SEQ ID NO: 2 DNA sequence of human DAPK |
| ccccggccggcgtgggtgtggggcgagtgggtgtgtgcggggtgtgcgcggtagagcgcgccagcgagcccggagcgcggagctggaggagc agcgagcgccgcgcagaacccgcagcgccggcctggcagggcagctcggaggtgggtgggccgcgccgccagcccgcttgcagggtccccattg gccgcctgccggccgccctccgcccaaaggcggcaaggagccgagaggctgcttcggagtgtgaggaggacagccggaccgagccaacgccg gggactttgttccctccgcggaggggactcggcaactcgcagcggcagggtctggggccggcgcctgggagggatctgcgcccccactcactccct agctgtgttcccgccgccgccccggctagtctccggcgctggcgcctatggtcggcctccgacagcgctccggagggaccgggggagctcccaggc gcccgggtgagtagccaggcgcggctccccggtcccccgacccccggcgccagcttttgctttcccagccagggcgcggtggggtttgtccgggca gtgcctcgagcaactgggaaggccaaggcggagggaaacttggcttcggggagaagtgcgatcgcagccgggaggcttccccagccccgcgggc cgggtgagaacaggtggcgccggcccgaccaggcgctttgtgtcggggcgcgaggatctggagcgaactgctgcgcctcggtgggccgctcccttc cctcccttgctcccccgggcggccgcacgccgggtcggccgggtaacggagagggagtcgccaggaatgtggctctggggactgcctcgctcggg gaaggggagagggtggcca |

| |
|---|
| SEQ ID NO: 3 DNA sequence of human PAX5 |
| aaacaaaaacccggcctgcgctcgtctaagcagcggggtttgcacatggagatgtcacaggccccgcgcacagcgcagaggccgcgacccccaa gcgcatgtcttaatagaaggtgcggctggaagacccgggctcccgggctccgcttcggtctgccccttcccgtaggtgcgctggctagcgcccggcgc aggctgaagccttccttccctccccccaaccccctataaaagtctggggcggcgcggcagcagcactgctgctctcccggcttcccgctctactccggcc gggccgggtccgccacgtctggcgcgctgagcaggcccggccgcgcagcgcctaccctttcctcgctccgggccggcagtgtggggcggcgcgctg ggggcgcggcgtg |

| |
|---|
| SEQ ID NO: 4 DNA sequence of human RASSF1A |
| ctgcgagagcgcgcccagccccgccttcgggccccacagtccctgcacccaggtttccattgcgcggctctcctcagctccttcccgccgcccagtctg gatcctgggggaggcgctgaagtcgggcccgccctgtggccccgcccggcccgcgcttgctagcgcccaaagccagcgaagcacgggcccaacc gggccatgtcgggggagcctgagctcattgagctgcgggagctggcacccgctgggcgcgctgggaagggccgcacccggctggagcgtgccaa cgcgctgcgcatcgcgcggggcaccgcgtgcaaccccacacggcagctggtccctggccgtggccaccgcttccagcccgcggggcccgccacgc |
| acacgtggtgcgacctctgtggcgacttcatctggggcgtcgtgcgcaaaggcctgcagt |

| |
|---|
| SEQ ID NO: 5 DNA sequence of human Reprimo |
| gagcgctcagcccggcacctgttcctccagcgccgccttcccacccctcggacccgcgccgctcgcggcgcccgcccgttcctgcgatgaatccg gccctaggcaaccagacggacgtggcgggcctgttcctggccaacagcagcgaggcgctggagcgagccgtgcgctgctgcacccaggcgtccgt ggtgaccgacgacggcttcgcggagggaggcccggacgagcgtagcctgtacataatgcgcgtggtgcagatcgcggtcatgtgcgtgctctcact caccgtggtcttcggcatcttcttcctcggctgcaatctgctcatcaagtccgagggcatgatcaacttcctcgtgaaggaccggaggccgtctaagga ggtggaggcggtggtcgtggggccctactgacccgccctctgcccccgcggcaaccgc |

| |
|---|
| SEQ ID NO: 6 DNA sequence of human RNF180 |
| ggacaagggagaccacagggataatttctgtggctctggtaaggggatgacaagggagaaaaactttcccacggttccgtctggcccgcggcgctt gtctgcctgcgcggggtcaaagcccggcgccgcccacgcgcggctcgggtgggaacccgcagacgtggggcgagcagggccgctggctgtggcg ggcgagcgccggggcgccacgtccgaggccgcggggtcggggctgcaggcacagctcgagcgctttccgcggggtttggctcctgtcgcttcccgt ctcgccgaaccggcatcgccgccgccggagccgcagcgagtcctcagagcctggctgctggcggccgggagcgccgggacggggcgcgaagcc ggaggctccgggacgtggatacaggtaaaggccggcgggtcggagtcgggcggggcgcgg |

| |
|---|
| SEQ ID NO: 7 DNA sequence of human RUNX3 |
| cctccccggccttcccctgcggcggcggcggcaagatggcgagaacagcggcgctgagcgcgcaggcggccgtggggcccggagggc gcgcccggcccgaggtgcgctcgatggtggacgtgctggcggaccacgcaggcgagctcgtgcgcaccgacagccccaacttcctctgctccgtgct gccctcgcactggcgctgcaacaagacgctgcccgtcgccttcaaggtgagtgcgggacccggggcggaggcgccggccctggggctccgggc gtctgagctgcgggagccagagcctcgggagcagtggggatgggaggtgcccgagacgccgcggcgacacccgggcacccggttcagtctggct gcgcgctcggctccgggacctcggcgttccgttttggatgcgccctgcaggaatgacttttaacggggttgccccgctccacctgggttttggggcgcc ttgcgtagagacgttggtgcggaaatggcggatggggttttgcgccccctaacccggatcgctcagatacagccctgcgggtaacggagaagag gatcccgggacaggggcaaggacaccgcgggtggggtgcgaacggtgaaaggcctaccctccgcctccagcacccctcctcccgcgccgcttcg caaaagcttcctgctcacaccctcgggcttccgaaattttaccggacggcgctaggccgggatcggggcactgctctccagacgtttgctcggggata ttatgcgtcccgaatggcgagttgagattggggaccccctaaccctggcagcccccagccatttagaggaccttttctttatgccagggagtaggcg actgtttcattttcatttttttaaggggcagcgag |

| |
|---|
| SEQ ID NO: 8 DNA sequence of human SDC2 |
| cggtgagcag agccggcgca gccacagcgc ggagccgcgg cgcccactgg tcctcggagc tgccaatcgg cgtgtaatcc tgtaggaatt tctcccgggt ttatctggga gtcacactgc cgcctcctct ccccagtcgc ccaggggagc ccggagaagc aggctcagga gggagggagc cagaggaaaa gaagaggagg agaaggagga ggacccgggg agggaggcgc ggcgcgggag gaggaggggc gcagccgcgg |
| agccagtggc cccgcttgga cgcgctgctc tccagatacc cccggagctc cagccgcgcg gatcgcgcgc tcccgccgct ctgcccctaa acttctgccg tagctccctt tcaagccagc gaatttattc cttaaaacca gaaactgaac ctcggcacgg gaaaggagtc cgcggaggag caaaaccaca gcagagcaag aagagcttca gagagcagcc ttcccggagc accaactccg tgtcgggagt gcagaaacca acaagtgaga gggcgccgcg ttcccggggc gcagctgcgg gcggcgggag caggcgcagg aggaggaagc gagcgcccccgagccccgag cccgagtccc cgagcctgag ccgcaatcgc tgcggtactc tgctccggat tcgtgtgcgc gggctgcgcc gagcgctggg caggaggctt cgttttgccc tggttgcaag cagcggctgg gagcagccgg tccctgggga atatgcggcg cgcgtggatc ctgctcacct tgggcttggt ggcctgcgtg tcggcggagt cggtgagtgg gccaggcg |

| |
|---|
| SEQ ID NO: 9 DNA sequence of human Septin9 |
| cggtgcgggt gcgggaacct gatccgcccg ggaggcgggg gcggggcggg ggcgcagcgc gcggggaggg gccggcgccc gccttcctcc cccattcatt cagctgagcc agggggccta ggggctcctc cggcggctag ctctgcactg caggagcgcg ggcgcggcgc cccagccagc gcgcagggcc cgggccccgc cgggggcgct tcctcgccgc tgccctccgc gcgacccgct gcccaccagc catcatgtcg gaccccgcgg tcaacgcgca gctggatggg atcatttcgg acttcgaagg tgggtgctgg gctggctgct gcggccgcgg acgtgctgga gaggaccctg cgggtgggcc tggcgcggga cgggggtgcg ctgaggggag acgggagtgc gctgagggga gacgggaccc ctaatccagg cgccctcccg ctgagagcgc cgcgcgcccc cggccccgtg cccgcgccgc ctacgtgggg gaccctgtta ggggcacccg cgtagaccct gcgcgccctc acaggaccct gtgctcgttc tgcgcactgc cg |

| |
|---|
| SEQ ID NO: 10 DNA sequence of human TCF4 |
| gaggtgttgagatttttttttttcccctcggggtgggtgcgaggggatgcatcctagcctgcccgacccggagcaagtcgcgtctccccgccggagc ccccccacccatttctttgctgaacttgcaattccgtgcgcctcggcgtgtttcccccctcccccttccctccgtcccctcccctccccggagaagagagtt ggtgttaagagtcagggatcttggctgtgtgtctgcggatctgtagtggcggcggcggcggcggcggggaggcagcaggcgcgggagcggg cgcaggagcaggcggcggcggtggcggcggcggttagacatgaacgccgcctcggcgccggcggtgcacggagagccccttctcgcgcgcgggc ggtaggtaccggcgcctgcggggctcggcggggcggaggcgcccggcggcgcggggttcgggctcggcggccccgcacgcggctccgcgcctcc cgcgccgcgggctcccggcgcccggcgctcccagaagagacaccccttcccctcccgccgcttccctcccctcgccgccagccccccgcccctcccc ttgatgccccctcggagggaccgaggactttgccaggggcctgactttaatttttataacccctttctttcacaaattagggtgctggacaattagagga cccgaccctccactccgctccccccaaccctgtca |

The term "biomarker gene or a fragment thereof" is often used herein when referring to the detection of a methylation level, because, in the choice of a template, as long as the length of the template is not less than the length of the region to be amplified, the primer pair used in the PCR amplification reaction does not distinguish between the entire gene or a fragment thereof (in fact, during the DNA extraction and subsequent bisulfite treatment, the gene is usually broken into fragments of different sizes).

In some preferred embodiments, the present invention uses the HeavyMethyl method to measure marker gene methylation. Therefore, in addition to the design of common Taqman primers, blocking primers are further designed. The nucleotide sequence of a blocking primer is designed to be paired with and bind to a template sequence in the region amplified by a corresponding primer pair. In addition, a chemical modification is introduced into a blocking primer at 3'-OH, which prevents the amplification with a DNA polymerase. The chemical modifications are, for example, C3 spacer (C3 Spacer), C6 spacer (C6 Spacer), inverted 3'end , 3' phosphate (3'P), etc. In embodiments of the method of the present invention, the nucleotide sequence of a blocking primer is designed to bind to an unmethylated template (treated with sulfite), but not to a methylated template (treated with sulfite). Therefore, when no methylation occurs in the region corresponding to a blocking primer, it can prevent the corresponding amplification reaction, and thereby improving the specificity of the detection method of the present invention.

In further preferred embodiments of the method of the present invention, it also comprises the use of fluorescent probes to monitor and/or quantify PCR amplification reactions in real time. The fluorescent report group at 5' end of a probe used may be FAM, JOE, TET, HEX, Cy3, Texas Red, Rox, or Cy5; the quenching group at the 3' end is BHQ1, BHQ2, BHQ3, TAMRA, DABCYL, or MGB.

The detection of the methylation levels of the biomarker gene(s) in the method of the present invention includes detecting whether there is/are methylation(s) in the biomarker gene, and quantitative and qualitative detection of the methylation(s).

The biological sample is selected from fluids or tissues extracted form the subject, and includes blood, serum, plasma, feces, lymph, cerebrospinal fluid, ascite, urine, tissue biopsy, etc., preferably plasma, serum and feces.

In the method of the present invention, the age of the subject can also be considered to predict the gastric cancer status in the subject.

In some embodiments, the method of the present invention further comprises the step of providing a written report or an electronic report on the gastric cancer prediction, and optionally, the report comprises a prediction about the presence or not or likelihood of a gastric cancer in the subject, or about the risk gradation of a gastric cancer in the subject.

In some embodiments, the method of the present invention also comprises establishing a report for a physician on the relative methylation levels of biomarker gene(s), and transmitting such report by post, fax, mailbox, etc. In one embodiment, a data stream containing the report of methylation levels of biomarker gene(s) is transmitted through the internet.

In some embodiments, a statistical method is used to construct a diagnostic model based on the methylation levels of the biomarker gene(s). The statistical method is selected from the following methods: multiple linear regression, lookup table, decision tree, support vector machine, Probit regression, logistic regression, cluster analysis, neighborhood analysis, genetic algorithm, Bayesian and non-Bayesian methods, etc.

In other embodiments, a prediction or diagnostic model based on the methylation levels of the biomarker gene(s) is provided. The model may be in the form of software code, a computer-readable format, or a written description for evaluating the relative methylation levels of the biomarker gene(s).

New and important additional information, which assists the physician in grading the risk of a patient suffering from a gastric cancer and planning the diagnostic steps to be taken next, can be obtained by using the method of the present invention. The method provided herein can similarly be used to assess the risk of a gastric cancer in an asymptomatic high-risk patient, and as a screening tool for the general population. It is contemplated that the method of the present invention can be used by a clinician as part of a comprehensive assessment of other predictive and diagnostic indicators.

The method of the present invention can be used to evaluate the therapeutic efficacies of existing chemotherapeutic agents, candidate chemotherapeutic agents and other types of cancer treatments. For example, biological samples can be taken from a subject before or after a treatment or during a treatment of the subject, and the methylation levels of the biomarker gene(s) can be detected as described above. The detection results are used to identify changes in the cancer status in the subject so as to determine the therapeutic efficacy.

The method of the invention can also be used to identify whether a subject is potentially developing a cancer. Relative methylation levels of the biomarker gene(s) in biological samples taken from a subject over time are detected, and the changes in the methylation levels of the biomarkers that point to the characteristics of a cancer are interpreted as a progress toward the cancer.

The combination of the biomarker genes provides a sensitive, specific and accurate means for predicting the presence of a gastric cancer or detecting a gastric cancer in different stages of the gastric cancer progression. Evaluation of the methylation levels in the biological sample may also be correlated with the presence of a pre-malignant or pre-clinical disorder in a patient. Therefore, the disclosed method can be used to predict or detect the presence of a gastric cancer in a sample, the stage of a gastric cancer, the subtype of a gastric cancer, the benignity or malignancy of a gastric cancer, the possibility of metastasis of a gastric cancer, the histological type of a neoplasm associated with a gastric cancer, the painlessness or aggressiveness of a cancer, and other gastric cancer characteristics related to the prevention, diagnosis, characterization, and treatment of a gastric cancer in a patient.

The method of the present invention can also be used to evaluate the effectiveness of candidate drugs to inhibit gastric cancer, evaluate the efficacy of gastric cancer therapy, monitor the progress of gastric cancer, select agents or therapies to inhibit gastric cancer, monitor the treatment of gastric cancer patients, monitor the inhibition status of gastric cancer in patients, and test the methylation levels of biomarker genes in animals after exposure to test compounds to assess the carcinogenic potential of the test compounds.

The present invention also provides a kit for detecting the gastric cancer status. In some embodiments, the kit may include a DNA extraction reagent and a bisulfite reagent. The DNA extraction reagent may include a lysis buffer, a binding buffer, a washing buffer, and an elution buffer. The lysis buffer is usually composed of a protein denaturant, a detergent, a pH buffering agent and a nuclease inhibitor. The binding buffer is usually composed of a protein denaturant and a pH buffer agent. The washing buffer is divided into washing buffer A and washing buffer B: washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is usually composed of a nuclease inhibitor and a pH buffering agent. The protein denaturant is selected from one or more of guanidine isothiocyanate, guanidine hydrochloride and urea; the detergent is selected from one or more of Tween20, IGEPAL CA-630, Triton X-100, NP-40 and SDS; the pH buffering agent is selected from one or more of Tris, boric acid, phosphate, MES and HEPES; the nuclease inhibitor is selected from one or more of EDTA, EGTA and DEPC. The bisulfite reagents include a bisulfite buffer and a protective buffer, in which the bisulfite salt is selected from one or more of sodium metabisulphite, sodium sulfite, sodium bisulfite, ammonium bisulfite and ammonium sulfite; the protection buffer is composed of an oxygen radical scavenger, and the oxygen radical scavenger is selected from one or more of hydroquinone, vitamin E, vitamin E derivatives, Trolox, trihydroxybenzoic acid and trihydroxybenzoic acid derivatives.

The kit of the present invention comprises a primer pair or primer pairs for methylation-specific PCR amplification reaction(s) for one or more of CDH1, DAPK, PAX5, RASSF1A, Reprimo, RNF180, RUNX3, SDC2, Septin9 and TCF4 gene. These primer pairs respectively detect the methylation of at least one nucleotide sequence in the nucleotide sequence of a target region of the corresponding gene.

The kit of the present invention may further comprise blocking primers and probes used in combination with the above-mentioned primer pairs (these blocking primers and probes are described above and below).

In certain embodiments, the kit may further comprise an instruction for using the kit to extract DNA from a biological sample and treating the DNA with the bisulfite reagent. In other embodiments, the kit further comprises an instruction for using the reagents in the kit to measure a biomarker level in the subject. In still other embodiments, the kit comprises an instruction for using the kit to determine the gastric cancer status in a subject.

The present invention also protects the method for detecting the methylation levels of the biomarker genes or fragments thereof with the kit. The method comprises the steps: extracting DNA in a biological sample by using the DNA extraction reagents, treating the extracted DNA with the bisulfite reagents, and using the treated DNA as a template to detect the methylation levels of the biomarker genes with the provided primer pairs.

The measurement method for the methylation level of a biomarker gene may be selected from one or more of the following methods: real-time fluorescent PCR, digital PCR, bisulfite sequencing, methylation-specific PCR, restriction enzyme analysis, high-resolution dissolution curve technology, gene chip technology and time-of-flight mass spectrometry.

The present invention is further described by the following examples.

### Example 1: DNA extraction

The DNA extraction reagent is composed of a lysis buffer, a binding buffer, a washing buffer, and an elution buffer. The lysis buffer is composed of a protein denaturant, a detergent, a pH buffering agent and a nuclease inhibitor. The binding buffer is composed of a protein denaturant and a pH buffering agent. The washing buffer is divided into washing buffer A and washing buffer B. Washing buffer A is composed of a protein denaturant, a nuclease inhibitor, a detergent, a pH buffering agent and ethanol; washing buffer B is composed of a nuclease inhibitor, a pH buffering agent and ethanol. The elution buffer is composed of a nuclease inhibitor and a pH buffering agent. The protein denaturant is guanidine hydrochloride; the detergent is Tween20; the pH buffering agent is Tris-HCl; and the nuclease inhibitor is EDTA.

In this example, a plasma sample of a gastric cancer patient is taken as an example to extract plasma DNA. The extraction method comprises the following steps:
(1) provide 1 mL plasma, add the same volume of the lysis buffer, then add proteinase K and Carrier RNA to achieve a final concentration of 100 mg/L and 1 µg/mL, mix by shaking, and incubate at 55°C for 30 min;
(2) add 100µL magnetic beads (purchased from Life technologies, catalog No: 37002D), and incubate for 1 hour with shaking;
(3) adsorbe the magnetic beads with a magnetic separator, and discard the supernatant solution;
(4) add 1mL of the washing buffer A to resuspend the magnetic beads and wash for 1min with shaking;
(5) adsorb the magnetic beads with the magnetic separator and discard the supernatant;
(6) add 1mL of the washing buffer B to resuspend the magnetic beads and wash for 1min with shaking;
(7) adsorb the magnetic beads with the magnetic separator and discard the supernatant solution;
(8) quickly centrifuge at 10,000 rpm for 1 minute, absorb the magnetic beads with the magnetic separator, and remove the residual supernatant solution;
(9) place the centrifuge tube loaded with the magnetic beads on a 55°C metal bath, and dry it for 10 min, with the lid open;
(10) add 100 µL of the elution buffer to resuspend the magnetic beads, place it on a 65°C metal bath, and elute for 10 min with shaking;
(11) adsorb the magnetic beads with the magnetic separator, take out the buffer containing the target DNA, quantify the DNA, and make a mark;
(12) store the eluted DNA in a refrigerator at 4°C for later use, or in a refrigerator at -20°C for long-term storage.

### Example 2: treatment of DNA with bisulfite

Treatment of DNA with bisulfite is to treat the extracted DNA sample with the bisulfite reagent. The bisulfite reagent is composed of a bisulfite buffer and a protection buffer. The bisulfite buffer is a mixed liquid of sodium bisulfite and water; the protection buffer is a mixed liquid of oxygen radical scavenger hydroquinone and water.

The DNA extracted in Example 1 is used as the processing object in this Example, and the DNA is treated with bisulfite. The steps comprise:
(1) prepare the bisulfite buffer: weigh 1 g of sodium bisulfite powder, and add water to it to obtain 3 M buffer solution;
(2) prepare the protection buffer: weigh 1 g of hydroquinone reagent, and add water to it to obtain 0.5M protection buffer;
(3) mix together 100 µL of the DNA solution, 200 µl of the bisulfite buffer and 50 µl of the protection solution, and mix by shaking;
(4) thermal treatment: 95°C for 5 minutes, 80°C for 60 minutes, and 4°C for 10 minutes;
(5) add 1 mL of the DNA binding buffer to the bisulfite-treated DNA solution, add 50 µL magnetic beads, and incubate for 1 hour with shaking;
(6) adsorbe the magnetic beads with a magnetic separator, and discard the supernatant solution;
(7) add 0.5mL of the washing buffer A to resuspend the magnetic beads and wash for 1minute with shaking;
(8) adsorbe the magnetic beads with the magnetic separator, and discard the supernatant;
(9) add 0.5mL of the washing buffer B to resuspend the magnetic beads and wash for 1min with shaking;
(10) adsorbe the magnetic beads with the magnetic separator, and discard the supernatant;
(11) quickly centrifuge at 10,000 rpm for 1 minute, absorb the magnetic beads with the magnetic separator, and remove the residual supernatant solution;
(12) place the centrifuge tube loaded with the magnetic beads on a 55°C metal bath, and dry it for 10 minutes, with the lid open;
(13) add 50 µLof the elution buffer to resuspend the magnetic beads, place it on a 65°C metal bath, and elute for 10 minutes with shaking;
(14) adsorb the magnetic beads with the magnetic separator, take out the buffer containing the target DNA, quantify the DNA, and make a mark.

### Example 3: Real-time fluorescent PCR detection of DNA methylation and verification of primer sets

In this example, a real-time fluorescent PCR was used as an example to detect the methylation levels of biomarker genes. The genes to be detected were CDH1, DAPK, PAX5, RASSF1A, Reprimo, RNF180, RUNX3, SDC2, Septin9 and TCF4 genes, and the internal reference gene was ACTB. In this example, the bisulfite-treated DNA of Example 2 was used as a template for real-time fluorescent PCR amplification. The DNA samples to be detected, a negative quality control product, a positive quality control product and no template controls were all detected in three replicates. The negative quality control product and the positive quality control product were respectively prepared as follows: take 400 µL of human leukocyte DNA with a concentration of 10 ng/µL and add it to TE buffer solution containing 1% BSA, mix, and dilute the solution to 200 mL to obtain a negative control substance with a concentration of 0.02 ng/µL; take 384 µL of human leukocyte DNA at a concentration of 10 ng/µL and 16 µL of Hela cell DNA at a concentration of 10 ng/µL, add them to TE buffer solution containing 1% BSA, mix, and dilute to 200 mL to obtain a concentration of 0.02 ng/µL, in which the positive DNA content is 4%.

For CDH1, DAPK, PAX5, RASSF1A, Reprimo, RNF180, RUNX3, SDC2, Septin9 and TCF4 genes, multiple sets of primer and probe combinations could be designed. However, the performance of each set of the probe and primer combinations may be different, so they needed to be verified through experiments.

Therefore, we designed a variety of primers and probes for CDH1, DAPK, PAX5, RASSF1A, Reprimo, RNF180, RUNX3, SDC2, Septin9 and TCF4 genes, which were respectively equivalent to, complementary to, or hybridizable to at least 15 consecutive nucleotides of the sequences as set forth in SEQ ID NOs: 1-10 or complementary sequences thereof, and verified the effectiveness of the designed primers and probes with methylated and unmethylated nucleic acid sequences as templates. We selected the following optimal primer sets and a primer set for the internal reference gene ACTB through real-time fluorescence PCR amplification results.
CDH1 primer set 1
   primer 1: SEQ ID NO 11: 5'- AGGGTTATCGCGTTTATGCG -3'
   primer 2: SEQ ID NO 12: 5'- CCACAACCAATCAACAAC -3'
   blocking primer: SEQ ID NO 13: 5'- TGTGTTTATGTGAGGTTGGGTGGGTG -C3-3'
   probe: SEQ ID NO 14: 5'- HEX- AAACGAAACTAACGACCCGCC -BHQ1-3'
   CDH1 primer set 2
   primer 1: SEQ ID NO 15: 5'- GTCGTTAGTTTCGTTTTG -3'
   primer 2: SEQ ID NO 16: 5'- CGTACCGCTAATTAACTAA -3'
   blocking primer: SEQ ID NO 17: 5'- TGTTAGTTTTGTTTTGGGGAGGGGTTTGTG -C3-3'
   probe: SEQ ID NO 18: 5'- HEX- ACCGACCACAACCAATCAACAAC -BHQ1-3'
DAPK primer set 1
   primer 1: SEQ ID NO 19: 5'- GGATAGTCGGATCGAGTT -3'
   primer 2: SEQ ID NO 20: 5'- GACCCCAAACCCTACC -3'
   blocking primer: SEQ ID NO 21: 5'-TGGATTGAGTTAATGTTGGGGATTTTGTTTTTTTTG -C3-3'
   probe: SEQ ID NO 22: 5'- Texas Red- TACGAATTACCGAATCCCCTCCG -BHQ2-3'
DAPK primer set 2
   primer 1: SEQ ID NO 23: 5'- CGGAGGGAAATTTGGTTTC -3'
   primer 2: SEQ ID NO 24: 5'- GACACAAAACGCCTAATC -3'
   blocking primer: SEQ ID NO 25: 5'- GGTTTTGGGGAGAAGTGTGATTGTAGTTG -C3-3'
   probe: SEQ ID NO 26: 5'- Texas Red- AACCGACGCCACCTATTCTCA -BHQ2-3'
PAX5 primer set 1
   primer 1: SEQ ID NO 27 5'- TAAAAATTCGGTTTGCGTTC -3'
   primer 2: SEQ ID NO 28: 5'- GACCCTCTACGCTATACG -3'
   blocking primer: SEQ ID NO 29: 5'- TGGTTTGTGTTTGTTTAAGTAGTGGGG -C3-3'
   probe: SEQ ID NO 30: 5'- Texas Red- ACATCTCCATATACAAACCCCGCTACT -BHQ2-3'
PAX5 primer set 2
   primer 1: SEQ ID NO 31: 5'- GGGTTCGTTACGTTTGG -3'
   primer 2: SEQ ID NO 32: 5'- CGACCCGAAACGAAAA -3'
   blocking primer: SEQ ID NO 33: 5'- TGTTATGTTTGGTGTGTTGAGTAGGTTTG -C3-3'
   probe: SEQ ID NO 34: 5'- Texas Red- CGACCGAACCTACTCAACGC -BHQ2-3'
RASSF1A primer set 1
   primer 1: SEQ ID NO 35: 5'- GCGTTGAAGTCGGGGTTCG -3'
   primer 2: SEQ ID NO 36: 5'- CCGATTAAACCCGTACTTC -3'
   blocking primer: SEQ ID NO 37: 5'- TTGGGGTTTGTTTTGTGGTTTCGTTTGGTTTGT -C3-3' probe: SEQ ID NO 38: 5'- FAM- CGCTAACAAACGCGAACCGA -BHQ1-3'
RASSF1A primer set 2
   primer 1: SEQ ID NO 39: 5'- GGGAGTTTGAGTTTATTGA -3'
   primer 2: SEQ ID NO 40: 5'- GATACGCAACGCGTTAACACG -3'
   blocking primer: SEQ ID NO 41: 5'- CACATTAACACACTCCAACCAAATACAACCCTT -C3-3' probe: SEQ ID NO 42: 5'- FAM- CGCCCAACGAATACCAACTCC -BHQ1-3'
Reprimo primer set 1
   primer 1: SEQ ID NO 43: 5'- GCGTTTAGTTCGGTATTTGTT -3'
   primer 2: SEQ ID NO 44: 5'- CGCAAAAACGAACGAACG -3'
   blocking primer: SEQ ID NO 45: 5'- TGTTTAGTTTGGTATTTGTTTTTTTAGTGTTGTTG -C3-3' probe: SEQ ID NO 46: 5'- Texas Red- CGCGAACGACGCGAATCCGAA -BHQ2-3'
Reprimo primer set 2
   primer 1: SEQ ID NO 47: 5'- TTTAGGTAATTAGACGGACG -3'
   primer 2: SEQ ID NO 48: 5'- ACGCCTAAATACAACAAC -3'
   blocking primer: SEQ ID NO 49: 5'- GATGGATGTGGTGGGTTTGTTTTTG -C3-3'
   probe: SEQ ID NO 50: 5'- Texas Red- TCCAACGCCTCGCTACTATTAACC -BHQ2-3'
RNF180 primer set 1
   primer 1: SEQ ID NO 51: 5'- TACGGTTTCGTTTGGTTCG -3'
   primer 2: SEQ ID NO 52: 5'- CACGTCTACGAATTCCCAC -3'
   blocking primer: SEQ ID NO 53 5'- TGTTTGGTTTGTGGTGTTTGTTTGTTTGTG -C3-3' probe: SEQ ID NO 54: 5'- JOE- CGTAAACGACGCCGAACTTTAACC -BHQ1-3'
RNF180 primer set 2
   primer 1: SEQ ID NO 55: 5'- GCGTTTGTTTGTTTGCG -3'
   primer 2: SEQ ID NO 56: 5'- TCTACGAATTCCCACCCG -3'
   blocking primer: SEQ ID NO 57 5'- TGTTTGGTTTGTGGTGTTTGTTTGTTTGTG -C3-3'
   probe: SEQ ID NO 58: 5'- JOE- CGTAAACGACGCCGAACTTTAACCC-BHQ1-3'
RNF180 primer set 3
   primer 1: SEQ ID NO 59: 5'- GGTCGTTGGTTGTGGC -3'
   primer 2: SEQ ID NO 60: 5'- GAACTATACCTACAACCCCG -3'
   blocking primer: SEQ ID NO 61 5'- TGTTGGTTGTGGTGGGTGAGTGTTG -C3-3'
   probe: SEQ ID NO 62: 5'- JOE- CCCGCGACCTCGAACGTAACG -BHQ1-3'
RUNX3 primer set 1
   primer 1: SEQ ID NO 63: 5'- TTCGGGTATTCGGTTTAG -3'
   primer 2: SEQ ID NO 64: 5'- CCTACAAAACGCATCCAA -3'
   blocking primer: SEQ ID NO 65: 5'- TGGGTATTTGGTTTAGTTTGGTTGTGTGTTTG -C3-3'
   probe: SEQ ID NO 66: 5'- HEX- CGAAACGCCGAAATCCCGAAA -BHQ1-3'
RUNX3 primer set 2
   primer 1: SEQ ID NO 67: 5'- CGTTGTAATAAGACGTTGTTC -3'
   primer 2: SEQ ID NO 68: 5'- CCACTACTCCCGAAACTC -3'
   blocking primer: SEQ ID NO 69: 5'- TGTTGTTTGTTGTTTTTAAGGTGAGTGTG -C3-3'
   probe: SEQ ID NO 70: 5'- HEX- AACTCCCGCAACTCAAACGC -BHQ1-3'
RUNX3 primer set 3
   primer 1: SEQ ID NO 71: 5'- CGCGTCGTTTCGTAAAAG -3'
   primer 2: SEQ ID NO 72: 5'- CCCGAACAAACGTCTAAA -3'
   blocking primer: SEQ ID NO 73: 5'- TGTTTTGTAAAAGTTTTTTGTTTATATTTTTGGGTTTTTG -C3-3' probe: SEQ ID NO 74: 5'- HEX- CGACCTAACGCCGTCCGATA -BHQ1-3'
SDC2 primer set 1
   primer 1: SEQ ID NO 75: 5'- CGGCGTAGTTATAGCGCGG -3'
   primer 2: SEQ ID NO 76: 5'- CCGAACTCCCCTAAACGACTAAA -3'
   blocking primer: SEQ ID NO 77: 5'- AGTTATAGTGTGGAGTTGTGGTGTTTATTGGTT -C3-3' probe: SEQ ID NO 78: 5'- FAM- TACAAAATTACACGCCGATTAACAACTCCG -BHQ1-3'
SDC2 primer set 2
   primer 1: SEQ ID NO 79: 5'- CGTAGGAGGAGGAAGCG -3'
   primer 2: SEQ ID NO 80: 5'- GCACACGAATCCGAAAC -3'
   blocking primer: SEQ ID NO 81: 5'- GGAGGAAGTGAGTGTTTTTGAGTTTTGAG -C3-3' probe: SEQ ID NO 82: 5'- FAM- AATACCGCAACGATTACGACTCAAACTCG -BHQ1-3'
SDC2 primer set 3
   primer 1: SEQ ID NO 83: 5'- CGAGTTTGAGTCGTAATCGTTG -3'
   primer 2: SEQ ID NO 84: 5'- CAACCAAAACAAAACGAAACC -3'
   blocking primer: SEQ ID NO 85: 5'- TGTAATTGTTGTGGTATTTTGTTTTGGATTTGTG -C3-3' probe: SEQ ID NO 86: 5'- FAM- AACGCTCGACGCAACCCGCGC -BHQ1-3'
Septin9 primer set 1
   primer 1: SEQ ID NO 87: 5'- CGCGATTCGTTGTTTATTAG-3'
   primer 2: SEQ ID NO 88: 5' - CACCTTCGAAATCCGAAA-3
   blocking primer: SEQ ID NO 89: 5' - AAAATCCAAAATAATCCCATCCAACTACACATTAAC -C3-3' probe: SEQ ID NO 90: 5' -FAM- CGCGTTAACCGCGAAATCCGACATAAT-BHQ1-3'
Septin9 primer set 2
   primer 1: SEQ ID NO 91: 5' - TAGCGTATTTTCGTTTCGC-3'
   primer 2: SEQ ID NO 92: 5' -CGAACTTCGAAAATAAATACTAAAC-3
   blocking primer: SEQ ID NO 93: 5' - TTTGTTTTGTGTTAGGTTTATTTGTAGGGTTT-C3-3' probe: SEQ ID NO 94: 5' -FAM-AACTACTACGACCGCGAACGTA-BHQ1-3'
TCF4 primer set 1
   primer 1: SEQ ID NO 95: 5'- GGCGGGGAGGTAGTAG -3'
   primer 2: SEQ ID NO 96: 5'- CCGAAACGACGTTCATATCTA -3'
   blocking primer: SEQ ID NO 97: 5'- TGGGGAGGTAGTAGGTGTGGGAGTG -C3-3' probe: SEQ ID NO 98: 5'-HEX- CTACTCCTACGCCCGCTCCC -BHQ1-3'
TCF4 primer set 2
   primer 1: SEQ ID NO 99: 5'- GTGTGTTTGCGGATTTGTA -3'
   primer 2: SEQ ID NO 100: 5'- ACGACGTTCATATCTAACC -3'
   blocking primer: SEQ ID NO 101: 5'- GTGGATTTGTAGTGGTGGTGGTGGTGGTG -C3-3' probe: SEQ ID NO 102: 5'-HEX- CTACTCCTACGCCCGCTCCC -BHQ1-3'
internal reference gene ACTB primer set
   primer 1: SEQ ID NO 103: 5'-GTGATGGAGGAGGTTTAGTAAGT-3'
   primer 2: SEQ ID NO 104: 5'-CCAATAAAACCTACTCCTCCCTT-3'
   probe: SEQ ID NO 105: 5'-Cy5-ACCACCACCCAACACACAATAACAAACACA-BHQ3-3'

All of the multiple sets of primers and probes could distinguish between methylated and unmethylated templates, and could be used as primers and probes to detect the methylations of CDH1, DAPK, PAX5, RASSF1A, Reprimo, RNF180, RUNX3, SDC2, Septin9 and TCF4 genes, respectively. Although the effectiveness of different primer and probe combinations were slightly different, the above primers and probes were suitable for the detection of methylations of CDH1, DAPK, PAX5, RASSF1A, Reprimo, RNF180, RUNX3, SDC2, Septin9 and TCF4 genes, respectively. Table 1 below showed the detection results of methylated and unmethylated templates (treated with bisulfite) of the above genes with various primer and probe combinations. Obviously, the designed primer and probe combinations were highly specific for the methylated templates.

**Table 1 detection results of the designed primer sets on methylated and unmethylated templates (Ct, mean)**

| | CDH1-1 | CDH1-2 | DAPK-1 | DAPK-2 | PAX5-1 | PAX5-2 | RASSF1A -1 | RASSF1A -2 | Reprimo-1 | Reprimo-2 |
|---|---|---|---|---|---|---|---|---|---|---|
| methylated DNA | 35.22 | 30.23 | 29.34 | 30.22 | 31.72 | 32.32 | 29.29 | 30.97 | 29.62 | 27.73 |
| unmethylated DNA | No Ct | No C1 | No Ct | No Ct | No Ct | No Ct | No Ct | No Ct | No Ct | No Ct |

| | RNF180 -1 | RNF180 -2 | RNF180 -3 | RUNX 3-1 | RUNX 3-2 | RUNX 3-3 | SDC2 -1 | SDC2 -2 | Septin9 -1 | Septin9 -2 | TCF4 -1 | TCF4 -2 |
|---|---|---|---|---|---|---|---|---|---|---|---|---|
| methylated DNA | 29.81 | 30.01 | 29.42 | 30.33 | 30.28 | 29.57 | 27.63 | 29.24 | 31.16 | 29.19 | 28.47 | 29.42 |
| unmethylated DNA | No Ct | No Ct | No Ct | No Ct | No Ct | No Ct | No Ct | No Ct | No Ct | No Ct | No Ct | No Ct |

Furthermore, we used DNAs from different cancer patients and healthy people as templates to further verify the effectiveness of the primer and probe combinations. DNAs in plasma samples from 5 cases of gastric cancer, 3 cases of liver cancer, and 5 cases of healthy persons were extracted by using the DNA extraction method of Example 1, and then DNA templates were treated with a bisulfite by using the method of Example 2. Using the above-mentioned multiple primer and probe sets, real-time fluorescent PCR experiments were performed. The Ct values of various marker genes in cancer samples and healthy person samples were measured respectively. The results were shown in Table 2.

**Table 2 detection results of the methylation levels of the specified genes in individuals with known gastric cancer status (including healthy individuals) with each primer set.**

| | CDH1-1 | CDH1-2 | DAPK-1 | DAPK-2 | PAX5-1 | PAX5-2 | RASSF1A -1 | RASSF1A -2 | Reprimo-1 | Reprimo-2 |
|---|---|---|---|---|---|---|---|---|---|---|
| GaCa 1 | 37.76 | 37.77 | 35.87 | 40.67 | 32.72 | 35.38 | 37.89 | 39.26 | 35.98 | 32.90 |
| GaCa 2 | 33.13 | 37.15 | 35.62 | 36.72 | 31.43 | 37.19 | 37.00 | 35.50 | 34.16 | 35.00 |
| GaCa 3 | 36.60 | 37.71 | 37.99 | 39.61 | 32.17 | 35.14 | 37.55 | 37.78 | 35.57 | 33.89 |
| GaCa 4 | 35.49 | 36.42 | 34.81 | 39.06 | 32.03 | 37.25 | 38.51 | 36.35 | 37.55 | 34.12 |
| GaCa 5 | 36.43 | 36.64 | 38.58 | 39.13 | 31.65 | 36.45 | 37.76 | 35.64 | 37.13 | 33.74 |
| HeCa 1 | No Ct | 43.61 | No Ct | 42.50 | No Ct | No Ct | 42.47 | No Ct | No Ct | 43.60 |
| HeCa 2 | No Ct | 43.27 | 43.92 | 42.23 | 42.67 | 43.66 | No Ct | No Ct | 43.66 | No Ct |
| HeCa 3 | 42.37 | No Ct | 44.99 | 43.95 | 42.99 | No Ct | No Ct | 42.52 | No Ct | No Ct |
| Con 1 | No Ct | No Ct | No Ct | No Ct | No Ct | No Ct | No Ct | No Ct | No Ct | 42.86 |
| Con 2 | No Ct | 44.06 | No Ct | No Ct | No Ct | 44.05 | No Ct | No Ct | No Ct | No Ct |
| Con 3 | No Ct | No Ct | No Ct | No Ct | No Ct | No Ct | No Ct | No Ct | No Ct | No Ct |
| Con 4 | No Ct | No Ct | No Ct | No Ct | No Ct | No Ct | No Ct | No Ct | No Ct | No Ct |
| Con 5 | No Ct | No Ct | No Ct | No Ct | No Ct | No Ct | No Ct | No Ct | No Ct | No Ct |

| | RNF180 -1 | RNF 180 -2 | RNF 180 -3 | RUNX 3-1 | RUNX 3-2 | RUNX 3-3 | SDC2-1 | SDC2-2 | Septin9 - 1 | Septin9-2 | TCF4-1 | TCF4-2 |
|---|---|---|---|---|---|---|---|---|---|---|---|---|
| GaCa 1 | 33.89 | 32.97 | 32.18 | 35.98 | 32.90 | 36.67 | 34.98 | 35.02 | 36.57 | 33.63 | 36.03 | 36.63 |
| GaCa 2 | 34.12 | 34.64 | 36.27 | 34.16 | 35.00 | 34.98 | 35.08 | 34.95 | 33.49 | 36.03 | 37.18 | 33.38 |
| GaCa 3 | 33.74 | 36.49 | 35.38 | 35,57 | 33.89 | 32.97 | 32.18 | 34.61 | 32.23 | 32.23 | 35.07 | 33.82 |
| GaCa 4 | 36.67 | 34.98 | 35.02 | 37.55 | 34.12 | 34.64 | 36.27 | 39.61 | 36.32 | 37.15 | 34.90 | 33.25 |
| GaCa 5 | 34.98 | 35.08 | 34.95 | 37.13 | 33.74 | 36.49 | 35.58 | 39.06 | 32.62 | 32.42 | 35.98 | 37.95 |
| HeCa 1 | No Ct | No Ct | No Ct | 44.07 | No Ct | 42.58 | No Ct | No Ct | No Ct | 43.46 | No Ct | 43.56 |
| HeCa 2 | No Ct | 42.52 | No Ct | 42.02 | 44.05 | No Ct | No Ct | 12.71 | 13.88 | 44.98 | No Cr | No Ct |
| HeCa 3 | 43.66 | No Ct | No Ct | 44.06 | No Ct | No Ct | 43.46 | No Ct | 44.48 | No Ct | No Ct | 42.25 |
| Con 1 | No Ct | 42.47 | No Ct | No Ct | No Ct | No Ct | No Ct | No Ct | No Ct | 43.56 | No Ct | No Ct |
| Con 2 | No Ct | No Ct | No Ct | No Ct | No Ct | No Ct | No Ct | No Ct | No Ct | No Ct | No Ct | No Ct |
| Con 3 | No Ct | Nc Ct | No Ct | No Ct | No Ct | No Ct | No Ct | No Ct | No Ct | No Ct | No Ct | No Ct |
| Con 4 | No Ct | No Ct | No Ct | No Ct | No Ct | No Ct | No Ct | No Ct | No Ct | No Ct | No Ct | No Ct |
| Con 5 | No Ct | No Ct | No Ct | No Ct | No Ct | No Ct | No Ct | No Ct | No Ct | No Ct | No Ct | No Ct |

| | | | | | | | | | | | | |
|---|---|---|---|---|---|---|---|---|---|---|---|---|
| **abbreviations: GaCa:gastric cancer; HeCa:liver cancer; Con:healthy** | | | | | | | | | | | | |

As can be seen from the above detected Ct values of the methylations of CDH1, DAPK, PAX5, RASSF1A, Reprimo, RNF180, RUNX3, SDC2, Septin9 and TCF4 genes, each of the above primer and probe combination generated a highly specific amplification for methylated DNA of gastric cancers, while there was no amplification or the Ct values of the amplifications were greater than 40 for other cancers or the healthy persons. Although the Ct values of the amplifications for gastric cancer samples with different combinations of primer pair and probe showed some differences, they were obviously different from those of other cancers and healthy person samples. Therefore, all of the above primer sets were suitable for gastric cancer detection.

### Example 4: sensitivity and specificity of the kit for detecting the plasmas of patients with gastric cancer or patients with benign disorder

203 samples from patients with pathologically identified gastric cancer and 261 samples from patients with pathologically identified benign disorder were used (see Table 3). All of the samples were collected from the Naval General Hospital of People's Liberation Army. The gastric cancer samples included all stages and common subtypes of the disease. The gastric cancer patients were confirmed by imaging and pathological diagnosis. The sample staging was based on international TNM staging standards, and the sample subtyping was determined according to tissue biopsies and immunohistochemical methods. Benign samples included common types of benign disorders founded in the whole study population. Complete clinical pathology reports were obtained after surgeries, including patient's age, smoking history, race, stage, subtype, and the collection sites were encoded for each sample.

**Table 3 gastric cancer stages and other characteristics of the collected samples**

| | **gastric cancer = stage and subtype** | | | | | **benign** |
|---|---|---|---|---|---|---|
| | **I** | **II** | **III** | **IV** | **total** | |
| **number of samples** | | | | | | **-** |
| **gastric cancer** | | | | | | **-** |
| **adenocarcinoma** | **11** | **28** | **94** | **46** | **179(88.2)** | **-** |
| **mucoid carcinoma** | **1** | **1** | **6** | **3** | **11(5.4)** | **-** |
| **undifferentiated carcinoma** | **1** | **1** | **2** | **1-2** | **6(3.0)** | **-** |
| **gastric cancer of other type** | **1** | **1** | **2** | **3** | **7(3.4)** | **-** |
| **sum** | **14** | **31** | **104** | **54** | **203** | **-** |
| | **(6.9)** | **(15.3)** | **(51.2)** | **(26.6)** | **(100)** | **-** |
| **benign** | | | | | | |
| **polyp** | **-** | **-** | **-** | **-** | **-** | **83(31.8)** |
| **gastric ulcer** | **-** | **-** | **-** | **-** | **-** | **89(34.1)** |
| **gastritis** | **-** | **-** | **-** | **-** | **-** | **68(26.1)** |
| **no abnormalities** | **-** | **-** | **-** | **-** | **-** | **21(8.0)** |
| **sum** | | | | | | **261(100)** |
| **ages of the polulation** | | | | | | |
| **median age (years)** | **54** | **59** | **58** | **60** | **58** | **49** |
| **age range (years)** | **26-85** | **23-86** | **27-91** | **25-88** | **23-91** | **18-85** |
| **mean age (years)** | **55.3** | **60.2** | **57.9** | **59.5** | **58.5** | **48.6** |
| **SD** | **11.4** | **10.3** | **8.5** | **10.7** | **8.2** | **7.6** |

DNAs were extracted from the samples by using the DNA extraction method of Example 1, the DNA templates were then treated with bisulfite by using the method of Example 2, and, next, real-time fluorescent PCR experiments were performed with the primer and probe combinations provided in Example 3 (for each biomarker gene, primer set 1 was used) to detect CDH1, DAPK, PAX5, RASSF1A, Reprimo, RNF180, RUNX3, SDC2, Septin9 and TCF4 genes and internal reference gene ACTB, and finally, the Ct values were obtained for each gene from samples of healthy persons and gastric cancer patients. As described in Example 3 above, the methylation levels of each gene could be indicated by these Ct values.

Commercially available software packages (IBM SPSS Statistics 24 and MedCalc 11.4.2.0, purchased from IBM and MedCalc, respectively) were used for descriptive statistics of plasma biomarker levels, receiver operating characteristic (ROC) curves and graphical displays. The nonparametric Kruskal-Wallis test (ANOVA) was used, and then a Dunn's multiple comparison post-test was used to determine statistical differences. For all statistical comparisons, p value < 0.05 was considered statistically significant.

The methylation levels of the above 10 marker genes were detected in plasmas from 203 patients with pathologically determined gastric cancer and 261 individuals with benign gastric disorders by real-time fluorescent PCR assays. To facilitate the determination of the ability of these biomarker genes to distinguish cancers from benign gastric disorders with similar symptoms, all samples were obtained from the same clinical population (based on patients undergone surgeries for gastric polyps). All samples were collected before any intervention and before the disease status was known. The disease status was then determined by pathological examination of *ex vivo* tissues. A single sample collection protocol was used to collect the plasmas and compliance was monitored. This ensured sample quality and eliminated any possibility of collection, processing and biological bias in the sample set. Normal healthy samples were not used in this study because they are usually more easily distinguishable than benign disorders. These samples showed that the average patient age among individuals with gastric cancers (58.5 years) was higher than that among individuals with benign disorders (48.6 years), and both increased with the progression of disease stages (Table 3). Overall, the distribution of gastric cancer subtypes was similar to that found in all gastric cancer cases in the population, with the proportion (88.2%) of adenocarcinomas being larger than that of other gastric cancers. The benign controls in the study represented common benign gastric diseases, including benign polyps, gastric ulcers, gastritis, etc.

For the detected data of the methylation level of each biomarker, MedCalc 11.4.2.0 software was used to generate a ROC curve and calculate the area under the curve (AUC) value with a 95% confidence interval. Compared with benign gastric disorders, the AUCs of the methylation levels of 10 biomarker genes in gastric cancer samples were all greater than 0.8 (*p* value < 0.05), and the AUCs were ranged from 0.809 to 0.903 (see Figure 1 and Table 4).

**Table 4 area under the curves (AUCs) though curve analysis of the receiver operating characteristic (ROC) curves of 10 marker genes**

| markers | AUC | standard error | 95% CI |
|---|---|---|---|
| CDH1 | 0.862 | 0.0283 | 0.806 - 0.906 |
| DAPK | 0.886 | 0.0252 | 0.833 - 0.926 |
| PAX5 | 0.828 | 0.0279 | 0.768 - 0.877 |
| RASSF1A | 0.903 | 0.0132 | 0.873 - 0.929 |
| Reprimo | 0.834 | 0.0184 | 0.797 - 0.867 |
| RNF180 | 0.891 | 0.0148 | 0.859 - 0.918 |
| RUNX3 | 0.809 | 0.0198 | 0.770 - 0.844 |
| SDC2 | 0.857 | 0.0255 | 0.801 - 0.902 |
| Septin9 | 0.900 | 0.0212 | 0.849 - 0.937 |
| TCF4 | 0.824 | 0.0186 | 0.786 - 0.858 |

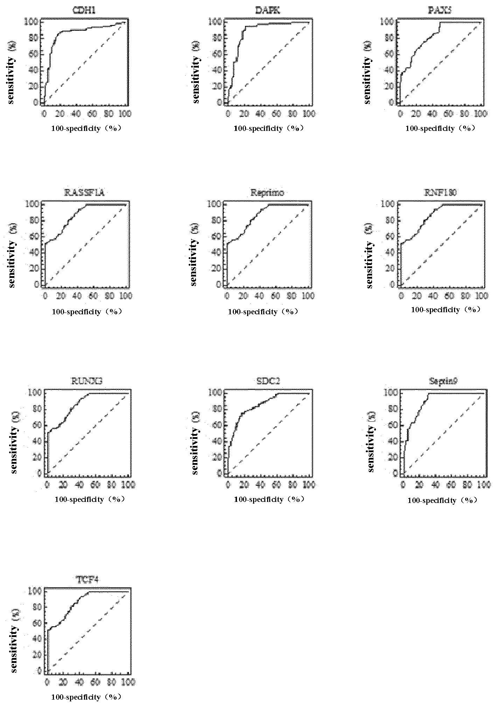

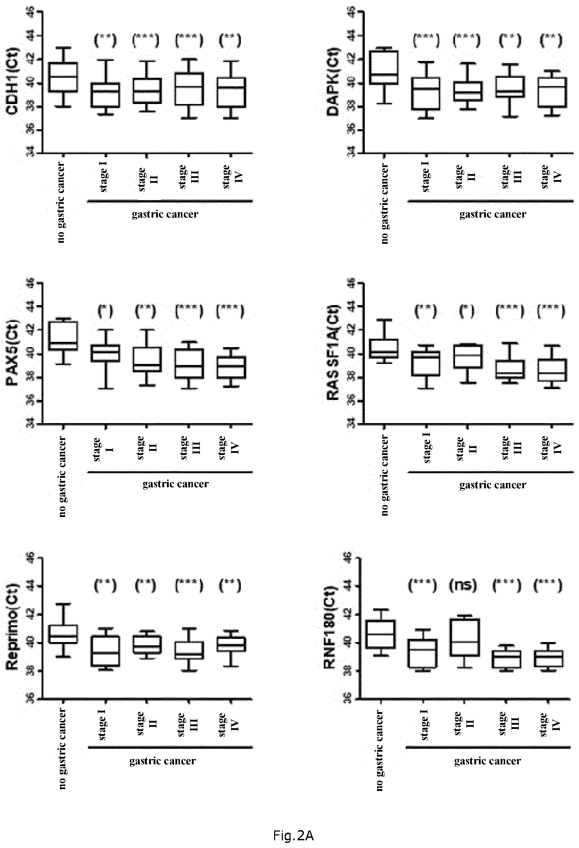

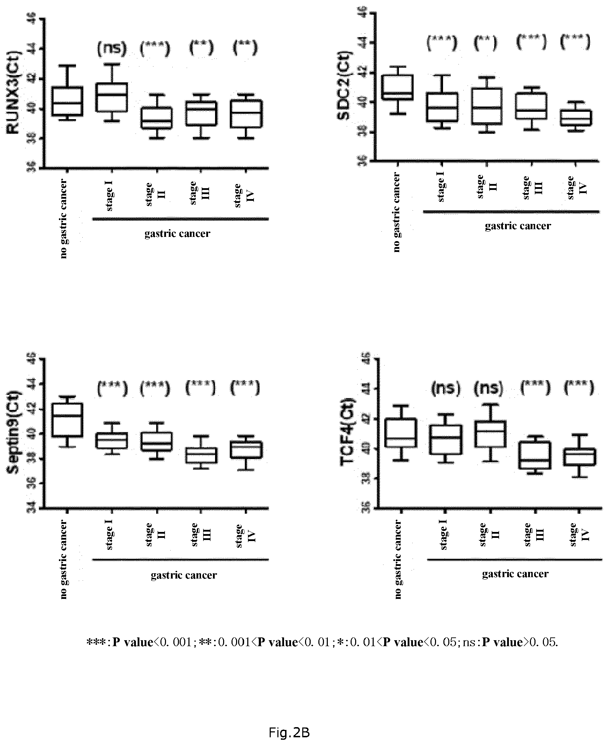

In order to determine whether certain biomarker genes could provide a better distinguishing ability between different stages of gastric cancers (especially in early stages), the distinguishing abilities of 10 biomarker genes (Figure 2) in stage I and stage II samples (the most important period for the marker detections) were compared. For stage I samples, DAPK, Septin9, RNF180 and SDC2 provided very high distinguishing abilities *(p* value <0.001), followed by CDH1, RASSF1A and Reprimo *(p* value 0.001 to 0.01) in descending order, and then PAX5 (*p* value 0.01 to 0.05). For RUNX3 and TCF4, there were no significant differences between stage I cancers and benign disorders (*p* value > 0.05). For stage II samples, in addition to CDH1 and RUNX3 (*p* value < 0.001), both DAPK and Septin9 again provided very high distinguishing abilities *(p* value < 0.001), and then PAX5, Reprimo and SDC2 *(p* value 0.001 to 0.01), and then RASSF1A *(p* value 0.01 to 0.05). There were no significant differences for RNF180 and TCF4 (*p* value > 0.05).

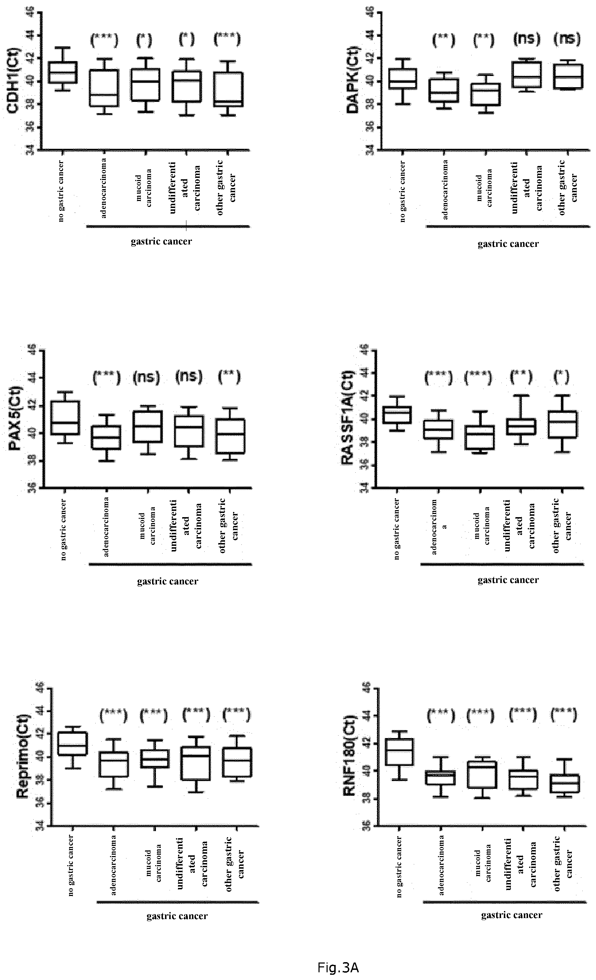

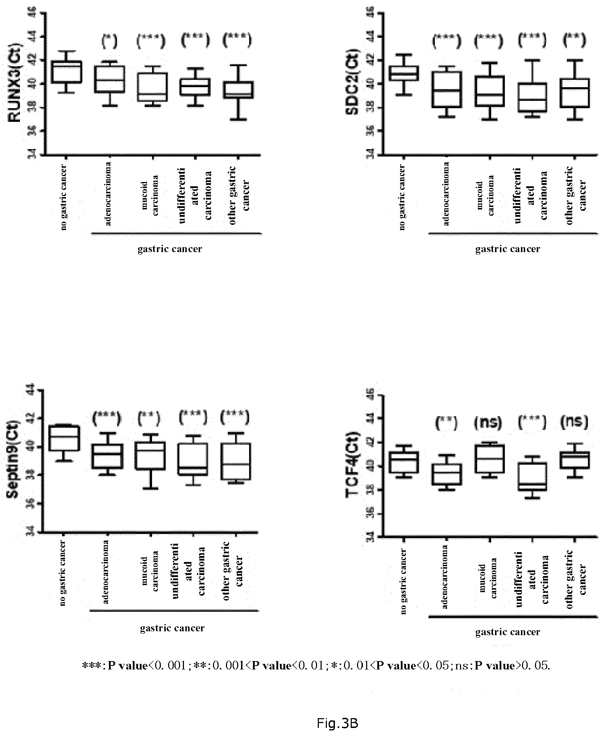

It was also evaluated whether there were statistically significant differences in the methylation levels of the above biomarker genes between samples from benign disorders and various subtypes of gastric cancers (Figure 3). For adenocarcinomas, CDH1, PAX5, RASSF1A, Reprimo, RNF180, SDC2 and Septin9 provided very high distinguishing abilities *(p* value < 0.001), followed by DAPK and TCF4 *(p* value 0.001 to 0.05), RUNX3 *(p* value 0.01 to 0.05) in descending order. For mucoid carcinomas, RASSF1A, Reprimo, RNF180, RUNX3 and SDC2 provided very high distinguishing abilities *(p* value <0.001), followed by DAPK and Septin9 *(p* value 0.001 to 0.05), CDH1 (*p* value 0.01 to 0.05) in descending order. For undifferentiated carcinomas, Reprimo, RNF180, RUNX3, SDC2, Septin9 and TCF4 provided very high distinguishing abilities (*p* value <0.001), followed by RASSF1A (*p* value 0.001 to 0.05), CDH1 (*p* value 0.01 to 0.05) in descending order. For other gastric cancers, CDH1, Reprimo, RNF180, RUNX3 and Septin9 provided very high distinguishing abilities (*p* value <0.001), followed by PAX5 and SDC2 *(p* value 0.001 to 0.05), RASSF1A *(p* value 0.01 to 0.05) in descending order.

In terms of simple operation and cost reduction, the detection of the methylation level of a single biomarker gene is better than the detection of the methylation levels of multiple biomarker genes. However, it is obvious that the methylation level of a single biomarker gene may not provide information on the inherent diversity of a complex disease, so it is often necessary to establish a diagnostic model with multiple markers. Multi-marker diagnosis model is established by using statistical analysis methods. The establishment of a diagnosis model with methylated gene markers for the detection of gastric cancers is described below by taking a logistic regression model as an example.

The training of the logistic regression model was conducted as follows: dividing the samples into cases and controls, and then optimizing the regression coefficients with IBM SPSS Statistics 24 software. Maximum likelihood of the data was trained with the logistic regression model by using one regression coefficient for each marker and one deviation parameter.

After training, the regression coefficient set defined the logistic regression model. By putting the methylation levels of the biomarkers into the logistic regression equation, those skilled in the art can easily use such diagnostic model to predict the possibility of any new sample to be identified as a case or a control.

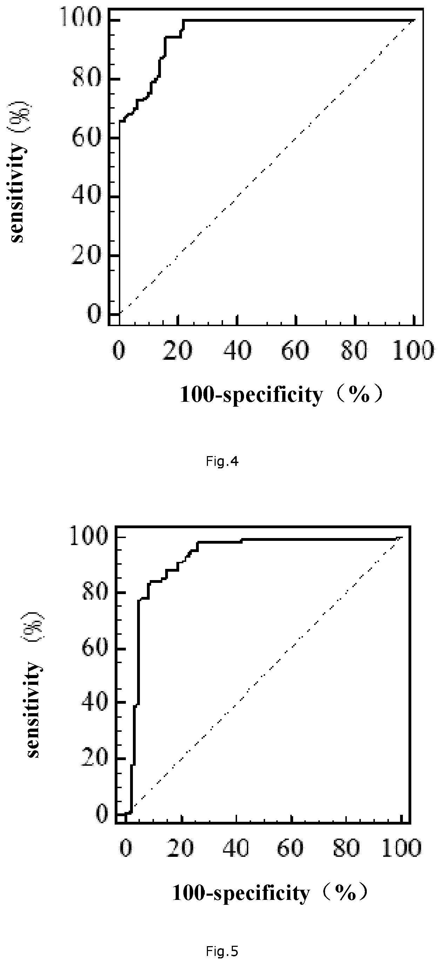

The AUCs of the methylation levels of the above 10 marker genes were all greater than 0.80. Next, we used the logistic regression to combine the 10 marker genes, which generated an AUC of 0.956 (standard error: 0.0121; 95% CI: 0.917-0.980; *p* value < 0.0001) (Figure 4). In order to simplify the monitoring and analysis method, the five markers with larger AUC values were combined and used to establish a logistic regression model. The obtained AUC value was 0.924 (standard error: 0.0214; 95% CI: 0.878-0.956; *p* value: < 0.0001) (Figure 5). For this sample set, a 98.0% sensitivity is acquired at a specificity of 65.2%. Two models were further compared by determining a model's sensitivity at a fixed specificity value and a model's specificity at a fixed sensitivity value (Table 5 and Table 6). For example, it could be selected that, when the sensitivity of the method was greater than about 95%, the sum of its sensitivity and specificity was greater than about 160%; or when the specificity of the method was greater than about 95%, the sum of its sensitivity and specificity was greater than about 165%. Generally, the sensitivity and specificity of a logistic regression model with 10 markers were slightly better than that with 5 markers. However, when the operational analysis procedures and cost were taken into consideration, the combination of the 5 markers may also be a good choice.

**Table 5 sensitivities at important specificity thresholds in logistic regression models of the 5 most characteristic marker genes and of the 10 marker genes**

| **specificity thresholds (%)** | **sensitivity (%)** | |
|---|---|---|
| | **5 markers** | **10 markers** |
| **80** | **91.2** | **94.2** |
| **85** | **87.1** | **88.5** |
| **90** | **84.3** | **74.0** |
| **95** | **76.2** | **69.0** |
| **100** | **49.6** | **65.0** |

**Table 6 specificities at important sensitivity thresholds in logistic regression models of the 5 most characteristic marker genes and of the 10 marker genes**

| **sensitivity thresholds (%)** | **specificity (%)** | |
|---|---|---|
| | **5 markers** | **10 markers** |
| **80** | **90.0** | **87.1** |
| **85** | **85.2** | **86.2** |
| **90** | **81.6** | **84.4** |
| **95** | **75.7** | **79.3** |
| **100** | **58.0** | **77.1** |

It should be noted that the detection results of the methylation levels provided in this Example were obtained with primer set 1 for each biomarker gene (for example, for CDH1 gene, use CDH1 primer set 1; for DAPK gene, use DAPK primer set 1, and so on), and similar detection results were obtained with other primer sets provided herein (data not shown).

The technical solutions provided by the present invention, through jointly detecting the methylation levels of one or more genes of CDH1, DAPK, PAX5, RASSF1A, Reprimo, RNF180, RUNX3, SDC2, Septin9 and TCF4 genes or fragments thereof, improved the sensitivity and specificity of gastric cancer detection, and thus ensured the accuracy and reliability of the test results. Moreover, the detection of methylatation of above biomarker genes in a sample with the primers provided in the kit of the present invention was able to quickly and conveniently determine the sample was positive or not and the risk value by using a logistic regression equation analysis.

The above Examples are only used to illustrate the technical solutions of the present invention and not to limit them.

## Claims

1. A method for identifying a gastric cancer status in a subject comprising following steps:
1) collecting a biological sample obtained from the subject;
2) detecting methylation levels of biomarker genes in the biological sample, wherein the biomarker genes comprise CDH1, DAPK, RASSF1A, RNF180, and Septin9; and
3) comparing the methylation levels detected in step 2) with normal methylation levels of the corresponding biomarker genes in a population to determine the gastric cancer status in the subject,
wherein the detection of the methylation level of the CDH1 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the CDH1 gene or a fragment thereof which is bisulfite-treated in the biological sample as a template;
the detection of the methylation level of the DAPK gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the DAPK gene or a fragment thereof which is bisulfite-treated in the biological sample as a template;
the detection of the methylation level of the RASSF1A gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the RASSF 1A gene or a fragment thereof which is bisulfite-treated in the biological sample as a template;
the detection of the methylation level of the RNF180 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the RNF180 gene or a fragment thereof which is bisulfite-treated in the biological sample as a template; and
the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the Septin9 gene or a fragment thereof which is bisulfite-treated in the biological sample as a template.

2. The method of claim 1, wherein step 2) comprises extracting DNA from the biological sample and treating the extracted DNA with a bisulfite, so that unmethylated cytosine residues in the DNA are deaminated, and methylated cytosine residues remain unchanged.

3. The method of claim 1, wherein in step 2) the biomarker genes are CDH1, DAPK, PAX5, RASSF1A, Reprimo, RNF180, RUNX3, SDC2, Septin9 and TCF4.

4. The method of claim 1, wherein step 2) comprises detecting the methylation levels of a target region within the biomarker genes, and wherein the target region is a nucleotide sequence of at least 15 bases in the biomarker genes, or a complementary sequence thereof.

5. The method of claim 3, wherein, in step 2),
the detection of the methylation level of the CDH1 gene comprises the use of a primer pair having the sequences as set forth in SEQ ID NOs: 11 and 12 or a primer pair having the sequences as set forth in SEQ ID NOs: 15 and 16 to carry out a PCR amplification reaction, with the CDH1 gene or a fragment thereof which is bisulfite-treated in the biological sample as a template;
the detection of the methylation level of the DAPK gene comprises the use of a primer pair having the sequences as set forth in SEQ ID NOs: 19 and 20 or a primer pair having the sequences as set forth in SEQ ID NOs: 23 and 24 to carry out a PCR amplification reaction, with the DAPK gene or a fragment thereof which is bisulfite-treated in the biological sample as a template;
the detection of the methylation level of the PAX5 gene comprises the use of a primer pair having the sequences as set forth in SEQ ID NOs: 27 and 28 or a primer pair having the sequences as set forth in SEQ ID NOs: 31 and 32 to carry out a PCR amplification reaction, with the PAX5 gene or a fragment thereof which is bisulfite-treated in the biological sample as a template;
the detection of the methylation level of the RASSF 1A gene comprises the use of a primer pair having the sequences as set forth in SEQ ID NOs: 35 and 36 or a primer pair having the sequences as set forth in SEQ ID NOs: 39 and 40 to carry out a PCR amplification reaction, with the RASSF 1A gene or a fragment thereof which is bisulfite-treated in the biological sample as a template;
the detection of the methylation level of the Reprimo gene comprises the use of a primer pair having the sequences as set forth in SEQ ID NOs: 43 and 44 or a primer pair having the sequences as set forth in SEQ ID NOs: 47 and 48 to carry out a PCR amplification reaction, with the Reprimo gene or a fragment thereof which is bisulfite-treated in the biological sample as a template;
the detection of the methylation level of the RNF180 gene comprises the use of a primer pair having the sequences as set forth in SEQ ID NOs: 51 and 52, a primer pair having the sequences as set forth in SEQ ID NOs: 55 and 56 or a primer pair having the sequences as set forth in SEQ ID NOs: 59 and 60 to carry out a PCR amplification reaction, with the RNF180 gene or a fragment thereof which is bisulfite-treated in the biological sample as a template;
the detection of the methylation level of the RUNX3 gene comprises the use of a primer pair having the sequences as set forth in SEQ ID NOs: 63 and 64, a primer pair having the sequences as set forth in SEQ ID NOs: 67 and 68 or a primer pair having the sequences as set forth in SEQ ID NOs: 71 and 72 to carry out a PCR amplification reaction, with the RUNX3 gene or a fragment thereof which is bisulfite-treated in the biological sample as a template;
the detection of the methylation level of the SDC2 gene comprises the use of a primer pair having the sequences as set forth in SEQ ID NOs: 75 and 76, a primer pair having the sequences as set forth in SEQ ID NOs: 79 and 80 or a primer pair having the sequences as set forth in SEQ ID NOs: 83 and 84 to carry out a PCR amplification reaction, with the SDC2 gene or a fragment thereof which is bisulfite-treated in the biological sample as a template;
the detection of the methylation level of the Septin9 gene comprises the use of a primer pair having the sequences as set forth in SEQ ID NOs: 87 and 88 or a primer pair having the sequences as set forth in SEQ ID NOs: 91 and 92 to carry out a PCR amplification reaction, with the Septin9 gene or a fragment thereof which is bisulfite-treated in the biological sample as a template; and
the detection of the methylation level of the TCF4 gene comprises the use of a primer pair having the sequences as set forth in SEQ ID NOs: 95 and 96 or a primer pair having the sequences as set forth in SEQ ID NOs: 99 and 100 to carry out a PCR amplification reaction, with the TCF4 gene or a fragment thereof which is bisulfite-treated in the biological sample as a template.

6. The method of claim 5, wherein, in step 2),
the detection of the methylation level of the CDH1 gene comprises the use of a primer pair having the sequences as set forth in SEQ ID NOs: 11 and 12 and a blocking primer having the sequence as set forth in SEQ ID NO: 13, or a primer pair having the sequences as set forth in SEQ ID NOs: 15 and 16 and a blocking primer having the sequence as set forth in SEQ ID NO: 17 to carry out a PCR amplification reaction, with the bisulfite-treated CDH1 gene or a fragment thereof in the biological sample as a template;
the detection of the methylation level of the DAPK gene comprises the use of a primer pair having the sequences as set forth in SEQ ID NOs: 19 and 20 and a blocking primer having the sequence as set forth in SEQ ID NO: 21, or a primer pair having the sequences as set forth in SEQ ID NOs: 23 and 24 and a blocking primer having the sequence as set forth in SEQ ID NO: 25 to carry out a PCR amplification reaction, with the bisulfite-treated DAPK gene or a fragment thereof in the biological sample as a template;
the detection of the methylation level of the PAX5 gene comprises the use of a primer pair having the sequences as set forth in SEQ ID NOs: 27 and 28 and a blocking primer having the sequence as set forth in SEQ ID NO: 29, or a primer pair having the sequences as set forth in SEQ ID NOs: 31 and 32 and a blocking primer having the sequence as set forth in SEQ ID NO: 33 to carry out a PCR amplification reaction, with the bisulfite-treated PAX5 gene or a fragment thereof in the biological sample as a template;
the detection of the methylation level of the RASSF 1A gene comprises the use of a primer pair having the sequences as set forth in SEQ ID NOs: 35 and 36 and a blocking primer having the sequence as set forth in SEQ ID NO: 37, or a primer pair having the sequences as set forth in SEQ ID NOs: 39 and 40 and a blocking primer having the sequence as set forth in SEQ ID NO: 41 to carry out a PCR amplification reaction, with the bisulfite-treated RASSF1A gene or a fragment thereof in the biological sample as a template;
the detection of the methylation level of the Reprimo gene comprises the use of a primer pair having the sequences as set forth in SEQ ID NOs: 43 and 44 and a blocking primer having the sequence as set forth in SEQ ID NO: 45, or a primer pair having the sequences as set forth in SEQ ID NOs: 47 and 48 and a blocking primer having the sequence as set forth in SEQ ID NO: 49 to carry out a PCR amplification reaction, with the bisulfite-treated Reprimo gene or a fragment thereof in the biological sample as a template;
the detection of the methylation level of the RNF180 gene comprises the use of a primer pair having the sequences as set forth in SEQ ID NOs: 51 and 52 and a blocking primer having the sequence as set forth in SEQ ID NO: 53, a primer pair having the sequences as set forth in SEQ ID NOs: 55 and 56 and a blocking primer having the sequence as set forth in SEQ ID NO: 57,or a primer pair having the sequences as set forth in SEQ ID NOs: 59 and 60 and a blocking primer having the sequence as set forth in SEQ ID NO: 61 to carry out a PCR amplification reaction, with the bisulfite-treated RNF180 gene or a fragment thereof in the biological sample as a template;
the detection of the methylation level of the RUNX3 gene comprises the use of a primer pair having the sequences as set forth in SEQ ID NOs: 63 and 64 and a blocking primer having the sequence as set forth in SEQ ID NO: 65, a primer pair having the sequences as set forth in SEQ ID NOs: 67 and 68 and a blocking primer having the sequence as set forth in SEQ ID NO: 69, or a primer pair having the sequences as set forth in SEQ ID NOs: 71 and 72 and a blocking primer having the sequence as set forth in SEQ ID NO: 73 to carry out a PCR amplification reaction, with the bisulfite-treated RUNX3 gene or a fragment thereof in the biological sample as a template;
the detection of the methylation level of the SDC2 gene comprises the use of a primer pair having the sequences as set forth in SEQ ID NOs: 75 and 76 and a blocking primer having the sequence as set forth in SEQ ID NO: 77, a primer pair having the sequences as set forth in SEQ ID NOs: 79 and 80 and a blocking primer having the sequence as set forth in SEQ ID NO: 81, or a primer pair having the sequences as set forth in SEQ ID NOs: 83 and 84 and a blocking primer having the sequence as set forth in SEQ ID NO: 85 to carry out a PCR amplification reaction, with the bisulfite-treated SDC2 gene or a fragment thereof in the biological sample as a template;
the detection of the methylation level of the Septin9 gene comprises the use of a primer pair having the sequences as set forth in SEQ ID NOs: 87 and 88 and a blocking primer having the sequence as set forth in SEQ ID NO: 89, or a primer pair having the sequences as set forth in SEQ ID NOs: 91 and 92 and a blocking primer having the sequence as set forth in SEQ ID NO: 93 to carry out a PCR amplification reaction, with the bisulfite-treated Septin9 gene or a fragment thereof in the biological sample as a template; and
the detection of the methylation level of the TCF4 gene comprises the use of a primer pair having the sequences as set forth in SEQ ID NOs: 95 and 96 and a blocking primer having the sequence as set forth in SEQ ID NO: 97, or a primer pair having the sequences as set forth in SEQ ID NOs: 99 and 100 and a blocking primer having the sequence as set forth in SEQ ID NO: 101 to carry out a PCR amplification reaction, with the bisulfite-treated TCF4 gene or a fragment thereof in the biological sample as a template,
wherein the blocking primers have a 3' end modification which prevents the extension and amplification of a DNA polymerase.

7. The method of claim 1, wherein step 3) comprises determining the gastric cancer status in the subject according to the methylation levels of the biomarker genes based on a logistic regression.

8. Use of a kit in a method of identifying a gastric cancer status in a subject comprising a primer pair for detecting methylation levels of a biomarker gene in a biological sample from the subject, wherein the primer pair is used to carry out a PCR amplification reaction with the biomarker gene or a fragment thereof which is bisulfite-treated as a template; and the biomarker genes comprise CDH1, DAPK, RASSF1A, RNF180, and Septin9,
wherein the primer pair used for the detection of the methylation level of CDH1 has the sequences as set forth in SEQ ID NOs: 11 and 12 or the sequences as set forth in SEQ ID NOs: 15 and 16;
the primer pair used for the detection of the methylation level of DAPK has the sequences as set forth in SEQ ID NOs: 19 and 20 or the sequences as set forth in SEQ ID NOs: 23 and 24;
the primer pair used for the detection of the methylation level of RASSF1A has the sequences as set forth in SEQ ID NOs: 35 and 36 or the sequences as set forth in SEQ ID NOs: 39 and 40;
the primer pair used for the detection of the methylation level of RNF180 has the sequences as set forth in SEQ ID NOs: 51 and 52, the sequences as set forth in SEQ ID NOs: 55 and 56 or the sequences as set forth in SEQ ID NOs: 59 and 60; and
the primer pair used for the detection of the methylation level of Septin9 has the sequences as set forth in SEQ ID NOs: 87 and 88 or the sequences as set forth in SEQ ID NOs: 91 and 92.

9. Use of a kit in a method of identifying a gastric cancer status of claim 8,
wherein the biomarker genes are CDH1, DAPK, PAX5, RASSF1A, Reprimo, RNF180, RUNX3, SDC2, Septin9 and TCF4.

10. Use of a kit in a method of identifying a gastric cancer status of claim 9,
wherein
the primer pair used for the detection of the methylation level of CDH1 has the sequences as set forth in SEQ ID NOs: 11 and 12 or the sequences as set forth in SEQ ID NOs: 15 and 16;
the primer pair used for the detection of the methylation level of DAPK has the sequences as set forth in SEQ ID NOs: 19 and 20 or the sequences as set forth in SEQ ID NOs: 23 and 24;
the primer pair used for the detection of the methylation level of PAX5 has the sequences as set forth in SEQ ID NOs: 27 and 28 or the sequences as set forth in SEQ ID NOs: 31 and 32;
the primer pair used for the detection of the methylation level of RASSF1A has the sequences as set forth in SEQ ID NOs: 35 and 36 or the sequences as set forth in SEQ ID NOs: 39 and 40;
the primer pair used for the detection of the methylation level of Reprimo has the sequences as set forth in SEQ ID NOs: 43 and 44 or has the sequences as set forth in SEQ ID NOs: 47 and 48;
the primer pair used for the detection of the methylation level of RNF180 has the sequences as set forth in SEQ ID NOs: 51 and 52, the sequences as set forth in SEQ ID NOs: 55 and 56 or the sequences as set forth in SEQ ID NOs: 59 and 60;
the primer pair used for the detection of the methylation level of RUNX3 has the sequences as set forth in SEQ ID NOs: 63 and 64, the sequences as set forth in SEQ ID NOs: 67 and 68, or the sequences as set forth in SEQ ID NOs: 71 and 72;
the primer pair used for the detection of the methylation level of SDC2 has the sequences as set forth in SEQ ID NOs: 75 and 76, the sequences as set forth in SEQ ID NOs: 79 and 80 or the sequences as set forth in SEQ ID NOs: 83 and 84;
the primer pair used for the detection of the methylation level of Septin9 has the sequences as set forth in SEQ ID NOs: 87 and 88 or the sequences as set forth in SEQ ID NOs: 91 and 92; and
the primer pair used for the detection of the methylation level of TCF4 has the sequences as set forth in SEQ ID NOs: 95 and 96 or the sequences as set forth in SEQ ID NOs: 99 and 100.

11. Use of a kit in a method of identifying a gastric cancer status of claim 10 further comprising a blocking primer, wherein
the blocking primer used in combination with the primer pair having the sequences as set forth in SEQ ID NO: 11 and 12 has the sequence as set forth in SEQ ID NO: 13;
the blocking primer used in combination with the primer pair having the sequences as set forth in SEQ ID NO: 15 and 16 has the sequence as set forth in SEQ ID NO: 17;
the blocking primer used in combination with the primer pair having the sequences as set forth in SEQ ID NO: 19 and 20 has the sequence as set forth in SEQ ID NO: 21;
the blocking primer used in combination with the primer pair having the sequences as set forth in SEQ ID NO: 23 and 24 has the sequence as set forth in SEQ ID NO: 25;
the blocking primer used in combination with the primer pair having the sequences as set forth in SEQ ID NO: 27 and 28 has the sequence as set forth in SEQ ID NO: 29;
the blocking primer used in combination with the primer pair having the sequences as set forth in SEQ ID NO: 31 and 32 has the sequence as set forth in SEQ ID NO: 33;
the blocking primer used in combination with the primer pair having the sequences as set forth in SEQ ID NO: 35 and 36 has the sequence as set forth in SEQ ID NO: 37;
the blocking primer used in combination with the primer pair having the sequences as set forth in SEQ ID NO: 39 and 40 has the sequence as set forth in SEQ ID NO: 41;
the blocking primer used in combination with the primer pair having the sequences as set forth in SEQ ID NO: 43 and 44 has the sequence as set forth in SEQ ID NO: 45;
the blocking primer used in combination with the primer pair having the sequences as set forth in SEQ ID NO: 47 and 48 has the sequence as set forth in SEQ ID NO: 49;
the blocking primer used in combination with the primer pair having the sequences as set forth in SEQ ID NO: 51 and 52 has the sequence as set forth in SEQ ID NO: 53;
the blocking primer used in combination with the primer pair having the sequences as set forth in SEQ ID NO: 55 and 56 has the sequence as set forth in SEQ ID NO: 57;
the blocking primer used in combination with the primer pair having the sequences as set forth in SEQ ID NO: 59 and 60 has the sequence as set forth in SEQ ID NO: 61;
the blocking primer used in combination with the primer pair having the sequences as set forth in SEQ ID NO: 63 and 64 has the sequence as set forth in SEQ ID NO: 65;
the blocking primer used in combination with the primer pair having the sequences as set forth in SEQ ID NO: 67 and 68 has the sequence as set forth in SEQ ID NO: 69;
the blocking primer used in combination with the primer pair having the sequences as set forth in SEQ ID NO: 71 and 72 has the sequence as set forth in SEQ ID NO: 73;
the blocking primer used in combination with the primer pair having the sequences as set forth in SEQ ID NO: 75 and 76 has the sequence as set forth in SEQ ID NO: 77;
the blocking primer used in combination with the primer pair having the sequences as set forth in SEQ ID NO: 79 and 80 has the sequence as set forth in SEQ ID NO: 81;
the blocking primer used in combination with the primer pair having the sequences as set forth in SEQ ID NO: 83 and 84 has the sequence as set forth in SEQ ID NO: 85;
the blocking primer used in combination with the primer pair having the sequences as set forth in SEQ ID NO: 87 and 88 has the sequence as set forth in SEQ ID NO: 89;
the blocking primer used in combination with the primer pair having the sequences as set forth in SEQ ID NO: 91 and 92 has the sequence as set forth in SEQ ID NO: 93;
the blocking primer used in combination with the primer pair having the sequences as set forth in SEQ ID NO: 95 and 96 has the sequence as set forth in SEQ ID NO: 97; and
the blocking primer used in combination with the primer pair having the sequences as set forth in SEQ ID NO: 99 and 100 has the sequence as set forth in SEQ ID NO: 101,
wherein the blocking primers have a 3' end modification which prevents the extension and amplification of a DNA polymerase.

12. Use of a kit in a method of identifying a gastric cancer status claim 8, further comprising a DNA extraction reagent and a bisulfite reagent.

13. Use of a kit in a method of identifying a gastric cancer status of claim 8, further comprising an instruction that describes how to use the kit and process detection results with a logistic regression.

## Patentansprüche

1. Verfahren zum Nachweis eines Magenkrebsstatus bei einer Testperson, umfassend die folgenden Schritte:
1) Bereitstellen einer der Testperson entnommenen biologischen Probe;
2) Nachweis der Methylierungsgrade von Biomarker-Genen in der biologischen Probe, wobei die Biomarker-Gene CDH1, DAPK, RASSF1A, RNF180 und Septin9 umfassen; und
3) Vergleichen der in Schritt 2) ermittelten Methylierungsgrade mit den normalen Methylierungsgraden der entsprechenden Biomarker-Gene in einer Population zum Bestimmmen des Magenkrebsstatus bei der Testperson,
wobei der Nachweis des Methylierungsgrades des CDH1-Gens die Verwendung eines Primer-Paares mit den in SEQ ID NOs: 11 und 12 angegebenen Sequenzen oder eines Primer-Paares mit den in SEQ ID NOs: 15 und 16 angegebenen Sequenzen umfasst zum Durchführen einer PCR-Amplifikationsreaktion, wobei das CDH1-Gen oder ein Bisulfit-behandeltes Fragment davon in der biologischen Probe als Template dient;
der Nachweis des Methylierungsgrades des DAPK-Gens die Verwendung eines Primer-Paares mit den in SEQ ID NOs: 19 und 20 angegebenen Sequenzen oder eines Primer-Paares mit den in SEQ ID NOs: 23 und 24 angegebenen Sequenzen umfasst zum Durchführen einer PCR-Amplifikationsreaktion, wobei das DAPK-Gen oder ein Bisulfit-behandeltes Fragment davon in der biologischen Probe als Template dient;
der Nachweis des Methylierungsgrades des RASSF1A-Gens die Verwendung eines Primer-Paares mit den in SEQ ID NOs: 35 und 36 oder eines Primer-Paares mit den in SEQ ID NOs: 39 und 40 angegebenen Sequenzen umfasst zum Durchführen einer PCR-Amplifikationsreaktion, wobei das RASSF1A-Gen oder ein Bisulfit-behandeltes Fragment davon in der biologischen Probe als Template dient;
der Nachweis des Methylierungsgrades des RNF180-Gens die Verwendung eines Primer-Paares mit den in den SEQ ID NOs: 51 und 52 angegebenen Sequenzen, eines Primer-Paares mit den in den SEQ ID NOs: 55 und 56 angegebenen Sequenzen oder eines Primer-Paares mit den in den SEQ ID NOs: 59 und 60 umfasst zum Durchführen einer PCR-Amplifikationsreaktion, wobei das RNF180-Gen oder ein Bisulfit-behandeltes Fragment davon in der biologischen Probe als Template dient; und
wobei der Nachweis des Methylierungsgrades des Septin9-Gens die Verwendung eines Primer-Paares mit den in SEQ ID NOs: 87 und 88 angegebenen Sequenzen oder eines Primer-Paares mit den in SEQ ID NOs: 91 und 92 angegebenen Sequenzen umfasst zum Durchführen einer PCR-Amplifikationsreaktion, wobei das Septin9-Gen oder ein Bisulfit-behandeltes Fragment davon in der biologischen Probe als Template dient.

2. Verfahren nach Anspruch 1, wobei Schritt 2) die Extraktion von DNA aus der biologischen Probe und die Behandlung der extrahierten DNA mit einem Bisulfit umfasst, so dass unmethylierte Cytosinreste in der DNA deaminiert werden und methylierte Cytosinreste unverändert bleiben.

3. Verfahren nach Anspruch 1, wobei in Schritt 2) die Biomarker-Gene CDH1, DAPK, PAX5, RASSF1A, Reprimo, RNF180, RUNX3, SDC2, Septin9 und TCF4 sind.

4. Verfahren nach Anspruch 1, wobei Schritt 2) das Nachweisen der Methylierungsgrade einer Zielregion innerhalb der Biomarker-Gene umfasst und wobei die Zielregion eine Nukleotidsequenz von mindestens 15 Basen in den Biomarker-Genen oder eine dazu komplementäre Sequenz ist.

5. Verfahren nach Anspruch 3, wobei in Schritt 2)
der Nachweis des Methylierungsgrades des CDH1-Gens die Verwendung eines Primer-Paares mit den in SEQ ID NOs: 11 und 12 angegebenen Sequenzen oder eines Primer-Paares mit den in SEQ ID NOs: 15 und 16 angegebenen Sequenzen umfasst zum Durchführen einer PCR-Amplifikationsreaktion, wobei das CDH1-Gen oder ein Bisulfit-behandeltes Fragment davon in der biologischen Probe als Template dient;
der Nachweis des Methylierungsgrades des DAPK-Gens die Verwendung eines Primer-Paares mit den in SEQ ID NOs: 19 und 20 angegebenen Sequenzen oder eines Primer-Paares mit den in SEQ ID NOs: 23 und 24 angegebenen Sequenzen umfasst zum Durchführen einer PCR-Amplifikationsreaktion, wobei das DAPK-Gen oder ein Bisulfit-behandeltes Fragment davon in der biologischen Probe als Template dient;
der Nachweis des Methylierungsgrades des PAX5-Gens die Verwendung eines Primer-Paares mit den in SEQ ID NOs: 27 und 28 angegebenen Sequenzen oder eines Primer-Paares mit den in SEQ ID NOs: 31 und 32 angegebenen Sequenzen umfasst zum Durchführen einer PCR-Amplifikationsreaktion, wobei das PAX5-Gen oder ein Bisulfit-behandeltes Fragment davon in der biologischen Probe als Template dient;
der Nachweis des Methylierungsgrades des RASSF1A-Gens die Verwendung eines Primer-Paares mit den in SEQ ID NOs: 35 und 36 oder eines Primer-Paares mit den in SEQ ID NOs: 39 und 40 angegebenen Sequenzen umfasst zum Durchführen einer PCR-Amplifikationsreaktion, wobei das RASSF1A-Gen oder ein Bisulfit-behandeltes Fragment davon in der biologischen Probe als Template dient;
der Nachweis des Methylierungsgrades des Reprimo-Gens die Verwendung eines Primer-Paares mit den in SEQ ID NOs: 43 und 44 angegebenen Sequenzen oder eines Primer-Paares mit den in SEQ ID NOs: 47 und 48 angegebenen Sequenzen umfasst zum Durchführen einer PCR-Amplifikationsreaktion, wobei das Reprimo-Gen oder ein Bisulfit-behandeltes Fragment davon in der biologischen Probe als Template dient;
der Nachweis des Methylierungsgrades des RNF180-Gens die Verwendung eines Primer-Paares mit den in SEQ ID NOs: 51 und 52 angegebenen Sequenzen, eines Primer-Paares mit den in SEQ ID NOs: 55 und 56 angegebenen Sequenzen oder eines Primer-Paares mit den in SEQ ID NOs: 59 und 60 umfasst zum Durchführen einer PCR-Amplifikationsreaktion, wobei das RNF180-Gen oder ein Bisulfit-behandeltes Fragment davon in der biologischen Probe als Template dient;
der Nachweis des Methylierungsgrades des RUNX3-Gens die Verwendung eines Primer-Paares mit den in SEQ ID NOs: 63 und 64 angegebenen Sequenzen, eines Primer-Paares mit den in SEQ ID NOs: 67 und 68 oder eines Primer-Paares mit den in den SEQ ID NOs: 71 und 72 angegebenen Sequenzen umfasst zum Durchführen einer PCR-Amplifikationsreaktion, wobei das RUNX3-Gen oder ein Bisulfit-behandeltes Fragment davon in der biologischen Probe als Template dient;
der Nachweis des Methylierungsgrades des SDC2-Gens die Verwendung eines Primer-Paares mit den in SEQ ID NOs: 75 und 76 angegebenen Sequenzen, eines Primer-Paares mit den in SEQ ID NOs: 79 und 80 angegebenen Sequenzen oder eines Primer-Paares mit den in SEQ ID NOs: 83 und 84 umfasst zum Durchführen einer PCR-Amplifikationsreaktion, wobei das SDC2-Gen oder ein Bisulfit-behandeltes Fragment davon in der biologischen Probe als Template dient;
der Nachweis des Methylierungsgrades des Septin9-Gens die Verwendung eines Primer-Paares mit den in SEQ ID NOs: 87 und 88 angegebenen Sequenzen oder eines Primer-Paares mit den in SEQ ID NOs: 91 und 92 angegebenen Sequenzen umfasst zum Durchführen einer PCR-Amplifikationsreaktion, wobei das Septin9-Gen oder ein Bisulfit-behandeltes Fragment davon in der biologischen Probe als Template dient; und
der Nachweis des Methylierungsgrades des TCF4-Gens die Verwendung eines Primer-Paares mit den in SEQ ID NOs: 95 und 96 angegebenen Sequenzen oder eines Primer-Paares mit den in SEQ ID NOs: 99 und 100 angegebenen Sequenzen umfasst zum Durchführen einer PCR-Amplifikationsreaktion, wobei das TCF4-Gen oder ein Bisulfit-behandeltes Fragment davon in der biologischen Probe als Template dient.

6. Verfahren nach Anspruch 5, wobei in Schritt 2)
der Nachweis des Methylierungsgrades des CDH1-Gens die Verwendung eines Primer-Paares mit den in SEQ ID NOs: 11 und 12 angegebenen Sequenzen und eines Blockierungsprimers mit der in SEQ ID NO: 13 angegebenen Sequenz oder eines Primer-Paares mit den in SEQ ID NOs: 15 und 16 angegebenen Sequenzen und eines Blockierungsprimers mit der in SEQ ID NO: 17 umfasst zum Durchführen einer PCR-Amplifikationsreaktion, wobei das Bisulfit-behandelte CDH1-Gen oder ein Fragment davon in der biologischen Probe als Template dient;
der Nachweis des Methylierungsgrades des DAPK-Gens die Verwendung eines Primer-Paares mit den in SEQ ID NOs: 19 und 20 angegebenen Sequenzen und eines Blockierungsprimers mit der in SEQ ID NO: 21 angegebenen Sequenz oder eines Primer-Paares mit den in SEQ ID NOs: 23 und 24 angegebenen Sequenzen und eines Blockierungsprimers mit der in SEQ ID NO: 25 umfasst zum Durchführen einer PCR-Amplifikationsreaktion, wobei das Bisulfit-behandelte DAPK-Gen oder ein Fragment davon in der biologischen Probe als Template dient;
der Nachweis des Methylierungsgrades des PAX5-Gens die Verwendung eines Primer-Paares mit den in den SEQ ID NOs: 27 und 28 angegebenen Sequenzen und eines Blockierungsprimers mit der in SEQ ID NO: 29 angegebenen Sequenz oder eines Primer-Paares mit den in den SEQ ID NOs: 31 und 32 angegebenen Sequenzen und eines Blockierungsprimers mit der in SEQ ID NO: 33 umfasst zum Durchführen einer PCR-Amplifikationsreaktion, wobei das Bisulfit-behandelte PAX5-Gen oder ein Fragment davon in der biologischen Probe als Template dient;
der Nachweis des Methylierungsgrades des RASSF1A-Gens die Verwendung eines Primer-Paares mit den in SEQ ID NOs: 35 und 36 angegebenen Sequenzen und eines Blockierungsprimers mit der in SEQ ID NO: 37 angegebenen Sequenz oder eines Primer-Paares mit den in SEQ ID NOs: 39 und 40 und eines Blockierungsprimers mit der in SEQ ID NO: 41 angegebenen Sequenz umfasst zum Durchführen einer PCR-Amplifikationsreaktion, wobei das mit Bisulfit-behandelte RASSF1A-Gen oder ein Fragment davon in der biologischen Probe als Template dient;
der Nachweis des Methylierungsgrades des Reprimo-Gens die Verwendung eines Primer-Paares mit den in SEQ ID NOs: 43 und 44 angegebenen Sequenzen und eines Blockierungsprimers mit der in SEQ ID NO: 45 angegebenen Sequenz oder eines Primer-Paares mit den in SEQ ID NOs: 47 und 48 angegebenen Sequenzen und eines Blockierungsprimers mit der in SEQ ID NO: 49 umfasst zum Durchführen einer PCR-Amplifikationsreaktion, wobei das Bisulfit-behandelte Reprimo-Gen oder ein Fragment davon in der biologischen Probe als Template dient;
der Nachweis des Methylierungsgrades des RNF180-Gens die Verwendung eines Primer-Paares mit den in SEQ ID NOs: 51 und 52 angegebenen Sequenzen und eines Blockierungsprimers mit der in SEQ ID NO: 53 angegebenen Sequenz, eines Primer-Paares mit den in SEQ ID NOs: 55 und 56 angegebenen Sequenzen und eines Blockierungsprimers mit der in SEQ ID NO: 57 angegebenen Sequenz, oder ein Primer-Paar mit den in SEQ ID NOs: 59 und 60 angegebenen Sequenzen und einen Blockierungsprimer mit der in SEQ ID NO: 61 angegebenen Sequenz umfasst zum Durchführen einer PCR-Amplifikationsreaktion, wobei das Bisulfit-behandelte RNF180-Gen oder ein Fragment davon in der biologischen Probe als Template dient;
der Nachweis des Methylierungsgrades des RUNX3-Gens die Verwendung eines Primer-Paares mit den in SEQ ID NOs: 63 und 64 angegebenen Sequenzen und eines Blockierungsprimers mit der in SEQ ID NO: 65 angegebenen Sequenz, eines Primer-Paares mit den in SEQ ID NOs: 67 und 68 angegebenen Sequenzen und eines Blockierungsprimers mit der in SEQ ID NO: 69 oder eines Primer-Paares mit den in SEQ ID NOs: 71 und 72 angegebenen Sequenzen und eines Blockierungsprimers mit der in SEQ ID NO: 73 angegebenen Sequenz umfasst zum Durchführen einer PCR-Amplifikationsreaktion, wobei das Bisulfit-behandelte RUNX3-Gen oder ein Fragment davon in der biologischen Probe als Template dient;
der Nachweis des Methylierungsgrades des SDC2-Gens die Verwendung eines Primer-Paares mit den in SEQ ID NOs: 75 und 76 angegebenen Sequenzen und eines Blockierungsprimers mit der in SEQ ID NO: 77 angegebenen Sequenz, eines Primer-Paares mit den in SEQ ID NOs: 79 und 80 angegebenen Sequenzen und eines Blockierungsprimers mit der in SEQ ID NO: 81 oder eines Primer-Paars mit den in SEQ ID NOs: 83 und 84 angegebenen Sequenzen und einen Blockierungsprimer mit der in SEQ ID NO: 85 angegebenen Sequenz umfasst zum Durchführen einer PCR-Amplifikationsreaktion, wobei das Bisulfit-behandelte SDC2-Gen oder ein Fragment davon in der biologischen Probe als Template dient;
der Nachweis des Methylierungsgrades des Septin9-Gens die Verwendung eines Primer-Paares mit den in SEQ ID NOs: 87 und 88 angegebenen Sequenzen und eines Blockierungsprimers mit der in SEQ ID NO: 89 angegebenen Sequenz oder eines Primer-Paares mit den in SEQ ID NOs: 91 und 92 angegebenen Sequenzen und eines Blockierungsprimers mit der in SEQ ID NO: 93 umfasst zum Durchführen einer PCR-Amplifikationsreaktion, wobei das Bisulfit-behandelte Septin9-Gen oder ein Fragment davon in der biologischen Probe als Template dient; und
wobei der Nachweis des Methylierungsgrades des TCF4-Gens die Verwendung eines Primer-Paares mit den in SEQ ID NOs: 95 und 96 angegebenen Sequenzen und eines Blockierungsprimers mit der in SEQ ID NO: 97 angegebenen Sequenz oder eines Primer-Paares mit den in SEQ ID NOs: 99 und 100 angegebenen Sequenzen und eines Blockierungsprimers mit der in SEQ ID NO: 101 umfasst zum Durchführen einer PCR-Amplifikationsreaktion, wobei das Bisulfit-behandelte TCF4-Gen oder ein Fragment davon in der biologischen Probe als Template dient,
wobei die Blockierungsprimer eine 3'-Endmodifikation aufweisen, die die Extension und Amplifikation durch eine DNA-Polymerase verhindert.

7. Verfahren nach Anspruch 1, wobei Schritt 3) der Nachweis des Status des Magenkrebses bei der Testperson anhand der Methylierungsgrade der Biomarker-Gene auf der Grundlage einer logistischen Regression umfasst.

8. Verwendung eines Kits in einem Verfahren zum Nachweis des Magenkrebsstatus bei einer Testperson, umfassend ein Primer-Paar zum Nachweis der Methylierungsgrade eines Biomarker-Gens in einer biologischen Probe der Testperson, wobei das Primer-Paar zur Durchführung einer PCR-Amplifikationsreaktion mit dem Biomarker-Gen oder ein Bisulfit-behandeltes Fragment davon als Template dient; und die Biomarker-Gene CDH1, DAPK, RASSF1A, RNF180 und Septin9,
wobei das zum Nachweis des Methylierungsgrades von CDH1 verwendete Primer-Paar die in SEQ ID NOs: 11 und 12 angegebenen Sequenzen oder die in SEQ ID NOs: 15 und 16 angegebenen Sequenzen aufweist;
das zum Nachweis des Methylierungsgrades von DAPK verwendete Primer-Paar die in SEQ ID NOs: 19 und 20 angegebenen Sequenzen oder die in SEQ ID NOs: 23 und 24 angegebenen Sequenzen aufweist;
das zum Nachweis des Methylierungsgrades von RASSF1A verwendete Primer-Paar die in SEQ ID NOs: 35 und 36 angegebenen Sequenzen oder die in SEQ ID NOs: 39 und 40 angegebenen Sequenzen aufweist;
das zum Nachweis des Methylierungsgrades von RNF180 verwendete Primer-Paar die in den SEQ ID NOs: 51 und 52 angegebenen Sequenzen, die in den SEQ ID NOs: 55 und 56 angegebenen Sequenzen oder die in den SEQ ID NOs: 59 und 60 angegebenen Sequenzen aufweist; und
das zum Nachweis des Methylierungsgrades von Septin9 verwendete Primer-Paar die in den SEQ ID NOs: 87 und 88 angegebenen Sequenzen oder die in den SEQ ID NOs: 91 und 92 angegebenen Sequenzen aufweist.

9. Verwendung eines Kits in einem Verfahren zum Nachweis des Magenkrebsstatus nach Anspruch 8, wobei die Biomarker-Gene CDH1, DAPK, PAX5, RASSF1A, Reprimo, RNF180, RUNX3, SDC2, Septin9 und TCF4 sind.

10. Verwendung eines Kits in einem Verfahren zum Nachweis des Magenkrebsstatus nach Anspruch 9, wobei
das zum Nachweis des Methylierungsgrades von CDH1 verwendete Primer-Paar die in SEQ ID NOs: 11 und 12 angegebenen Sequenzen oder die in SEQ ID NOs: 15 und 16 angegebenen Sequenzen aufweist;
das für den Nachweis des Methylierungsgrades von DAPK verwendete Primer-Paar die in SEQ ID NOs: 19 und 20 angegebenen Sequenzen oder die in SEQ ID NOs: 23 und 24 angegebenen Sequenzen aufweist;
das zum Nachweis des Methylierungsgrades von PAX5 verwendete Primer-Paar die in den SEQ ID NOs: 27 und 28 angegebenen Sequenzen oder die in den SEQ ID NOs: 31 und 32 angegebenen Sequenzen aufweist;
das zum Nachweis des Methylierungsgrades von RASSF1A verwendete Primer-Paar die in den SEQ ID NOs: 35 und 36 angegebenen Sequenzen oder die in den SEQ ID NOs: 39 und 40 angegebenen Sequenzen aufweist;
das zum Nachweis des Methylierungsgrades von Reprimo verwendete Primer-Paar die in den SEQ ID NOs: 43 und 44 angegebenen Sequenzen oder die in den SEQ ID NOs: 47 und 48 angegebenen Sequenzen aufweist;
das zum Nachweis des Methylierungsgrades von RNF180 verwendete Primer-Paar die in SEQ ID NOs: 51 und 52 angegebenen Sequenzen, die in SEQ ID NOs: 55 und 56 angegebenen Sequenzen oder die in SEQ ID NOs: 59 und 60 angegebenen Sequenzen aufweist;
das zum Nachweis des Methylierungsgrades von RUNX3 verwendete Primer-Paar die in den SEQ ID NOs: 63 und 64 angegebenen Sequenzen, die in den SEQ ID NOs: 67 und 68 angegebenen Sequenzen oder die in den SEQ ID NOs: 71 und 72 angegebenen Sequenzen aufweist;
das zum Nachweis des Methylierungsgrades von SDC2 verwendete Primer-Paar die in den SEQ ID NOs: 75 und 76 angegebenen Sequenzen, die in den SEQ ID NOs: 79 und 80 angegebenen Sequenzen oder die in den SEQ ID NOs: 83 und 84 angegebenen Sequenzen aufweist;
das zum Nachweis des Methylierungsgrades von Septin9 verwendete Primer-Paar die in SEQ ID NOs: 87 und 88 angegebenen Sequenzen oder die in SEQ ID NOs: 91 und 92 angegebenen Sequenzen aufweist; und
das zum Nachweis des Methylierungsgrades von TCF4 verwendete Primer-Paar die in den SEQ ID NOs: 95 und 96 angegebenen Sequenzen oder die in den SEQ ID NOs: 99 und 100 angegebenen Sequenzen aufweist.

11. Verwendung eines Kits in einem Verfahren zur Identifizierung des Magenkrebsstatus gemäß Anspruch 10, ferner umfassend einen Blockierungsprimer, wobei
der Blockierungsprimer, der in Kombination mit dem Primer-Paar mit den in SEQ ID NO: 11 und 12 angegebenen Sequenzen verwendet wird, die in SEQ ID NO: 13 angegebene Sequenz aufweist;
der Blockierungsprimer, der in Kombination mit dem Primer-Paar mit den in SEQ ID NO: 15 und 16 angegebenen Sequenzen verwendet wird, die in SEQ ID NO: 17 angegebene Sequenz aufweist;
der Blockierungsprimer, der in Kombination mit dem Primer-Paar mit den in SEQ ID NO: 19 und 20 angegebenen Sequenzen verwendet wird, die in SEQ ID NO: 21 angegebene Sequenz aufweist;
der Blockierungsprimer, der in Kombination mit dem Primer-Paar mit den in SEQ ID NO: 23 und 24 angegebenen Sequenzen verwendet wird, die in SEQ ID NO: 25 angegebene Sequenz aufweist;
der Blockierungsprimer, der in Kombination mit dem Primer-Paar mit den in SEQ ID NO: 27 und 28 angegebenen Sequenzen verwendet wird, die in SEQ ID NO: 29 angegebene Sequenz aufweist;
der Blockierungsprimer, der in Kombination mit dem Primer-Paar mit den in SEQ ID NO: 31 und 32 angegebenen Sequenzen verwendet wird, die in SEQ ID NO: 33 angegebene Sequenz aufweist;
der Blockierungsprimer, der in Kombination mit dem Primer-Paar mit den in SEQ ID NO: 35 und 36 angegebenen Sequenzen verwendet wird, die in SEQ ID NO: 37 angegebene Sequenz aufweist;
der Blockierungsprimer, der in Kombination mit dem Primer-Paar mit den in SEQ ID NO: 39 und 40 angegebenen Sequenzen verwendet wird, die in SEQ ID NO: 41 angegebene Sequenz aufweist;
der Blockierungsprimer, der in Kombination mit dem Primer-Paar mit den in SEQ ID NO: 43 und 44 angegebenen Sequenzen verwendet wird, die in SEQ ID NO: 45 angegebene Sequenz aufweist;
der Blockierungsprimer, der in Kombination mit dem Primer-Paar mit den in SEQ ID NO: 47 und 48 angegebenen Sequenzen verwendet wird, die in SEQ ID NO: 49 angegebene Sequenz aufweist;
der Blockierungsprimer, der in Kombination mit dem Primer-Paar mit den in SEQ ID NO: 51 und 52 angegebenen Sequenzen verwendet wird, die in SEQ ID NO: 53 angegebene Sequenz aufweist;
der Blockierungsprimer, der in Kombination mit dem Primer-Paar mit den in SEQ ID NO: 55 und 56 angegebenen Sequenzen verwendet wird, die in SEQ ID NO: 57 angegebene Sequenz aufweist;
der Blockierungsprimer, der in Kombination mit dem Primer-Paar mit den in SEQ ID NO: 59 und 60 angegebenen Sequenzen verwendet wird, die in SEQ ID NO: 61 angegebene Sequenz aufweist;
der Blockierungsprimer, der in Kombination mit dem Primer-Paar mit den in SEQ ID NO: 63 und 64 angegebenen Sequenzen verwendet wird, die in SEQ ID NO: 65 angegebene Sequenz aufweist;
der Blockierungsprimer, der in Kombination mit dem Primer-Paar mit den in SEQ ID NO: 67 und 68 angegebenen Sequenzen verwendet wird, die in SEQ ID NO: 69 angegebene Sequenz aufweist;
der Blockierungsprimer, der in Kombination mit dem Primer-Paar mit den in SEQ ID NO: 71 und 72 angegebenen Sequenzen verwendet wird, die in SEQ ID NO: 73 angegebene Sequenz aufweist;
der Blockierungsprimer, der in Kombination mit dem Primer-Paar mit den in SEQ ID NO: 75 und 76 angegebenen Sequenzen verwendet wird, die in SEQ ID NO: 77 angegebene Sequenz aufweist;
der Blockierungsprimer, der in Kombination mit dem Primer-Paar mit den in SEQ ID NO: 79 und 80 angegebenen Sequenzen verwendet wird, die in SEQ ID NO: 81 angegebene Sequenz aufweist;
der Blockierungsprimer, der in Kombination mit dem Primer-Paar mit den in SEQ ID NO: 83 und 84 angegebenen Sequenzen verwendet wird, die in SEQ ID NO: 85 angegebene Sequenz aufweist;
der Blockierungsprimer, der in Kombination mit dem Primer-Paar mit den in SEQ ID NO: 87 und 88 angegebenen Sequenzen verwendet wird, die in SEQ ID NO: 89 angegebene Sequenz aufweist;
der Blockierungsprimer, der in Kombination mit dem Primer-Paar mit den in SEQ ID NO: 91 und 92 angegebenen Sequenzen verwendet wird, die in SEQ ID NO: 93 angegebene Sequenz aufweist;
der Blockierungsprimer, der in Kombination mit dem Primer-Paar mit den in SEQ ID NO: 95 und 96 angegebenen Sequenzen verwendet wird, die in SEQ ID NO: 97 angegebene Sequenz aufweist; und
der Blockierungsprimer, der in Kombination mit dem Primer-Paar mit den in SEQ ID NO: 99 und 100 angegebenen Sequenzen verwendet wird, die in SEQ ID NO: 101 angegebene Sequenz aufweist,
wobei die Blockierungsprimer eine 3'-Endmodifikation aufweisen, die die Extension und Amplifikation durch eine DNA-Polymerase verhindert.

12. Verwendung eines Kits in einem Verfahren zum Nachweis des Status von Magenkrebs gemäß Anspruch 8, das ferner ein DNA-Extraktionsreagenz und ein Bisulfit-Reagenz umfasst.

13. Verwendung eines Kits in einem Verfahren zum Nachweis des Magenkrebsstatus gemäß Anspruch 8, umfassend ferner eine Anleitung, die beschreibt, wie das Kit zu verwenden ist und wie die Nachweisergebnisse mittels logistischer Regression zu verarbeiten sind.

## Revendications

1. Procédé d'identification d'un état d'un cancer gastrique chez un sujet, comprenant les étapes suivantes:
1) prélever un échantillon biologique provenant du sujet;
2) détecter des niveaux de méthylation de gènes de biomarqueurs dans l'échantillon biologique, les gènes de biomarqueurs comprenant CDH1, DAPK, RASSF1A, RNF180 et Septin9 ; et
3) comparer les niveaux de méthylation détectés à l'étape 2) avec des niveaux de méthylation normaux des gènes de biomarqueurs correspondants dans une population afin de déterminer l'état du cancer gastrique chez le sujet,
dans lequel la détection du niveau de méthylation du gène CDH1 comprend l'utilisation d'une paire d'amorces ayant les séquences décrites dans les SEQ ID NOs : 11 et 12 ou d'une paire d'amorces ayant les séquences décrites dans les SEQ ID NOs : 15 et 16 pour effectuer une réaction d'amplification par PCR, avec le gène CDH1 ou un fragment de celui-ci traité au bisulfite dans l'échantillon biologique comme une matrice;
la détection du niveau de méthylation du gène DAPK comprend l'utilisation d'une paire d'amorces ayant les séquences décrites dans les SEQ ID NOs : 19 et 20 ou d'une paire d'amorces ayant les séquences décrites dans les SEQ ID NOs : 23 et 24 pour effectuer une réaction d'amplification par PCR, avec le gène DAPK ou un fragment de celui-ci traité au bisulfite dans l'échantillon biologique comme une matrice;
la détection du niveau de méthylation du gène RASSF1A comprend l'utilisation d'une paire d'amorces ayant les séquences décrites dans les SEQ ID NOs : 35 et 36 ou d'une paire d'amorces ayant les séquences décrites dans les SEQ ID NOs : 39 et 40 pour effectuer une réaction d'amplification par PCR, avec le gène RASSF1A ou un fragment de celui-ci traité au bisulfite dans l'échantillon biologique comme matrice;
la détection du niveau de méthylation du gène RNF180 comprend l'utilisation d'une paire d'amorces ayant les séquences décrites dans les SEQ ID NOs : 51 et 52, d'une paire d'amorces ayant les séquences décrites dans les SEQ ID NOs : 55 et 56 ou d'une paire d'amorces ayant les séquences décrites dans les SEQ ID NOs : 59 et 60 pour effectuer une réaction d'amplification par PCR, avec le gène RNF180 ou un fragment de celui-ci traité au bisulfite dans l'échantillon d' s biologiques comme matrice; et
la détection du niveau de méthylation du gène Septin9 comprend l'utilisation d'une paire d'amorces ayant les séquences décrites dans les SEQ ID NOs : 87 et 88 ou d'une paire d'amorces ayant les séquences décrites dans les SEQ ID NOs : 91 et 92 pour effectuer une réaction d'amplification par PCR, avec le gène Septin9 ou un fragment de celui-ci traité au bisulfite dans l'échantillon biologique comme matrice.

2. Procédé selon la revendication 1, dans lequel l'étape 2) comprend l'extraction d'ADN de l'échantillon biologique et le traitement de l'ADN extrait avec un bisulfite, de sorte que les résidus de cytosine non méthylés dans l'ADN soient désaminés, et que les résidus de cytosine méthylés restent inchangés.

3. Procédé selon la revendication 1, dans lequel, à l'étape 2), les gènes biomarqueurs sont CDH1, DAPK, PAX5, RASSF1A, Reprimo, RNF180, RUNX3, SDC2, Septin9 et TCF4.

4. Procédé selon la revendication 1, dans lequel l'étape 2) comprend la détection des niveaux de méthylation d'une région cible au sein des gènes biomarqueurs, et dans lequel la région cible est une séquence nucléotidique d'au moins 15 bases dans les gènes biomarqueurs, ou une séquence complémentaire de celle-ci.

5. Procédé selon la revendication 3, dans lequel, à l'étape 2),
la détection du niveau de méthylation du gène CDH1 comprend l'utilisation d'une paire d'amorces ayant les séquences décrites dans les SEQ ID NOs : 11 et 12 ou d'une paire d'amorces ayant les séquences décrites dans les SEQ ID NOs : 15 et 16 pour effectuer une réaction d'amplification par PCR, avec le gène CDH1 ou un fragment de celui-ci traité au bisulfite dans l'échantillon biologique comme matrice;
la détection du niveau de méthylation du gène DAPK comprend l'utilisation d'une paire d'amorces ayant les séquences décrites dans les SEQ ID NOs : 19 et 20 ou d'une paire d'amorces ayant les séquences décrites dans les SEQ ID NOs : 23 et 24 pour effectuer une réaction d'amplification par PCR, avec le gène DAPK ou un fragment de celui-ci traité au bisulfite dans l'échantillon biologique comme matrice;
la détection du niveau de méthylation du gène PAX5 comprend l'utilisation d'une paire d'amorces ayant les séquences décrites dans les SEQ ID NOs : 27 et 28 ou d'une paire d'amorces ayant les séquences décrites dans les SEQ ID NOs : 31 et 32 pour effectuer une réaction d'amplification par PCR, avec le gène PAX5 ou un fragment de celui-ci traité au bisulfite dans l'échantillon biologique comme matrice;
la détection du niveau de méthylation du gène RASSF1A comprend l'utilisation d'une paire d'amorces ayant les séquences décrites dans les SEQ ID NOs : 35 et 36 ou d'une paire d'amorces ayant les séquences décrites dans les SEQ ID NOs : 39 et 40 pour effectuer une réaction d'amplification par PCR, avec le gène RASSF1A ou un fragment de celui-ci traité au bisulfite dans l'échantillon biologique comme matrice;
la détection du niveau de méthylation du gène Reprimo comprend l'utilisation d'une paire d'amorces ayant les séquences décrites dans les SEQ ID NOs : 43 et 44 ou d'une paire d'amorces ayant les séquences décrites dans les SEQ ID NOs : 47 et 48 pour effectuer une réaction d'amplification par PCR, avec le gène Reprimo ou un fragment de celui-ci traité au bisulfite dans l'échantillon biologique comme matrice;
la détection du niveau de méthylation du gène RNF180 comprend l'utilisation d'une paire d'amorces ayant les séquences décrites dans les SEQ ID NOs : 51 et 52, d'une paire d'amorces ayant les séquences décrites dans les SEQ ID NOs : 55 et 56 ou d'une paire d'amorces ayant les séquences décrites dans les SEQ ID NOs : 59 et 60 pour effectuer une réaction d'amplification par PCR, avec le gène RNF180 ou un fragment de celui-ci ayant traité au bisulfite dans l'échantillon biologique comme matrice;
la détection du niveau de méthylation du gène RUNX3 comprend l'utilisation d'une paire d'amorces ayant les séquences décrites dans les SEQ ID NOs : 63 et 64, d'une paire d'amorces ayant les séquences décrites dans les SEQ ID NOs : 67 et 68 ou d'une paire d'amorces ayant les séquences décrites dans les SEQ ID NOs : 71 et 72 pour effectuer une réaction d'amplification par PCR, avec le gène RUNX3 ou un fragment de celui-ci traité au bisulfite dans l'échantillon biologique comme matrice;
la détection du niveau de méthylation du gène SDC2 comprend l'utilisation d'une paire d'amorces ayant les séquences décrites dans les SEQ ID NOs : 75 et 76, d'une paire d'amorces ayant les séquences décrites dans les SEQ ID NOs : 79 et 80 ou d'une paire d'amorces ayant les séquences décrites dans les SEQ ID NOs : 83 et 84 pour effectuer une réaction d'amplification par PCR, avec le gène SDC2 ou un fragment de celui-ci traité au bisulfite dans l'échantillon biologique comme matrice;
la détection du niveau de méthylation du gène Septin9 comprend l'utilisation d'une paire d'amorces ayant les séquences décrites dans les SEQ ID NOs : 87 et 88 ou d'une paire d'amorces ayant les séquences décrites dans les SEQ ID NOs : 91 et 92 pour effectuer une réaction d'amplification par PCR, avec le gène Septin9 ou un fragment de celui-ci traité au bisulfite dans l'échantillon biologique comme matrice; et
la détection du niveau de méthylation du gène TCF4 comprend l'utilisation d'une paire d'amorces ayant les séquences décrites dans les SEQ ID NOs : 95 et 96 ou d'une paire d'amorces ayant les séquences décrites dans les SEQ ID NOs : 99 et 100 pour effectuer une réaction d'amplification par PCR, avec le gène TCF4 ou un fragment de celui-ci traité au bisulfite dans l'échantillon biologique comme matrice.

6. Procédé selon la revendication 5, dans lequel, à l'étape 2),
la détection du niveau de méthylation du gène CDH1 comprend l'utilisation d'une paire d'amorces ayant les séquences décrites dans les SEQ ID NOs : 11 et 12 et d'une amorce de blocage ayant la séquence décrite dans la SEQ ID NO : 13, ou d'une paire d'amorces ayant les séquences décrites dans les SEQ ID NOs : 15 et 16 et d'une amorce de blocage ayant la séquence décrite dans la SEQ ID NO : 17 pour effectuer une réaction d'amplification par PCR, avec le gène CDH1 traité au bisulfite ou un fragment de celui-ci présent dans l'échantillon biologique comme matrice;
la détection du niveau de méthylation du gène DAPK comprend l'utilisation d'une paire d'amorces ayant les séquences décrites dans les SEQ ID NO : 19 et 20 et d'une amorce de blocage ayant la séquence décrite dans la SEQ ID NO : 21, ou d'une paire d'amorces ayant les séquences décrites dans les SEQ ID NO : 23 et 24 et d'une amorce de blocage ayant la séquence décrite dans la SEQ ID NO : 25 pour effectuer une réaction d'amplification par PCR, avec le gène DAPK traité au bisulfite ou un fragment de celui-ci présent dans l'échantillon biologique comme matrice;
la détection du niveau de méthylation du gène PAX5 comprend l'utilisation d'une paire d'amorces ayant les séquences décrites dans les SEQ ID NO : 27 et 28 et d'une amorce de blocage ayant la séquence décrite dans la SEQ ID NO : 29, ou d'une paire d'amorces ayant les séquences décrites dans les SEQ ID NO : 31 et 32 et d'une amorce de blocage ayant la séquence décrite dans la SEQ ID NO : 33 pour effectuer une réaction d'amplification par PCR, avec le gène PAX5 traité au bisulfite ou un fragment de celui-ci présent dans l'échantillon biologique comme matrice;
la détection du niveau de méthylation du gène RASSF1A comprend l'utilisation d'une paire d'amorces ayant les séquences décrites dans les SEQ ID NOs : 35 et 36 et d'une amorce de blocage ayant la séquence décrite dans la SEQ ID NO : 37, ou d'une paire d'amorces ayant les séquences décrites dans les SEQ ID NOs : 39 et 40 et d'une amorce de blocage ayant la séquence décrite dans SEQ ID NO : 41 pour effectuer une réaction d'amplification par PCR, avec le gène RASSF1A traité au bisulfite ou un fragment de celui-ci présent dans l'échantillon biologique comme matrice;
la détection du niveau de méthylation du gène Reprimo comprend l'utilisation d'une paire d'amorces ayant les séquences décrites dans les SEQ ID NO : 43 et 44 et d'une amorce de blocage ayant la séquence décrite dans la SEQ ID NO : 45, ou d'une paire d'amorces ayant les séquences décrites dans les SEQ ID NO : 47 et 48 et d'une amorce de blocage ayant la séquence décrite dans la SEQ ID NO : 49 pour effectuer une réaction d'amplification par PCR, avec le gène Reprimo traité au bisulfite ou un fragment de celui-ci présent dans l'échantillon biologique comme matrice;
la détection du niveau de méthylation du gène RNF180 comprend l'utilisation d'une paire d'amorces ayant les séquences décrites dans les SEQ ID NO : 51 et 52 et d'une amorce de blocage ayant la séquence décrite dans la SEQ ID NO : 53, d'une paire d'amorces ayant les séquences décrites dans les SEQ ID NO : 55 et 56 et d'une amorce de blocage ayant la séquence décrite dans la SEQ ID NO : 57, ou une paire d'amorces ayant les séquences décrites dans les SEQ ID NO : 59 et 60 et une amorce de blocage ayant la séquence décrite dans la SEQ ID NO : 61 pour effectuer une réaction d'amplification par PCR, avec le gène RNF180 traité au bisulfite ou un fragment de celui-ci présent dans l'échantillon biologique comme matrice;
la détection du niveau de méthylation du gène RUNX3 comprend l'utilisation d'une paire d'amorces ayant les séquences décrites dans les SEQ ID NOs : 63 et 64 et d'une amorce de blocage ayant la séquence décrite dans la SEQ ID NO : 65, d'une paire d'amorces ayant les séquences décrites dans les SEQ ID NOs : 67 et 68 et d'une amorce de blocage ayant la séquence décrite dans la SEQ ID NO : 69, ou d'une paire d'amorces ayant les séquences décrites dans les SEQ ID NO : 71 et 72 et d'une amorce de blocage ayant la séquence décrite dans la SEQ ID NO : 73 pour effectuer une réaction d'amplification par PCR, avec le gène RUNX3 traité au bisulfite ou un fragment de celui-ci présent dans l'échantillon biologique comme matrice;
la détection du niveau de méthylation du gène SDC2 comprend l'utilisation d'une paire d'amorces ayant les séquences décrites dans les SEQ ID NO : 75 et 76 et d'une amorce de blocage ayant la séquence décrite dans la SEQ ID NO : 77, d'une paire d'amorces ayant les séquences décrites dans les SEQ ID NO : 79 et 80 et d'une amorce de blocage ayant la séquence décrite dans la SEQ ID NO : 81, ou d'une paire d'amorces ayant les séquences décrites dans les SEQ ID NO : 83 et 84 et d'une amorce de blocage ayant la séquence décrite dans la SEQ ID NO : 85 pour effectuer une réaction d'amplification par PCR, avec le gène SDC2 traité au bisulfite ou un fragment de celui-ci présent dans l'échantillon biologique comme matrice;
la détection du niveau de méthylation du gène Septin9 comprend l'utilisation d'une paire d'amorces ayant les séquences décrites dans les SEQ ID NO : 87 et 88 et d'une amorce de blocage ayant la séquence décrite dans la SEQ ID NO : 89, ou d'une paire d'amorces ayant les séquences décrites dans les SEQ ID NO : 91 et 92 et d'une amorce de blocage ayant la séquence décrite dans la SEQ ID NO : 93 pour réaliser une réaction d'amplification par PCR, avec le gène Septin9 traité au bisulfite ou un fragment de celui-ci présent dans l'échantillon biologique comme matrice; et
la détection du niveau de méthylation du gène TCF4 comprend l'utilisation d'une paire d'amorces ayant les séquences décrites dans les SEQ ID NO : 95 et 96 et d'une amorce de blocage ayant la séquence décrite dans la SEQ ID NO : 97, ou d'une paire d'amorces ayant les séquences décrites dans les SEQ ID NO : 99 et 100 et d'une amorce de blocage ayant la séquence décrite dans la SEQ ID NO : 101 pour effectuer une réaction d'amplification par PCR, avec le gène TCF4 traité au bisulfite ou un fragment de celui-ci présent dans l'échantillon biologique comme matrice,
dans laquelle les amorces de blocage présentent une modification à l'extrémité 3' qui empêche l'extension et l'amplification par une ADN polymérase.

7. Procédé selon la revendication 1, dans lequel l'étape 3) comprend déterminer l'état du cancer gastrique chez le sujet en fonction des niveaux de méthylation des gènes de biomarqueurs sur la base d'une régression logistique.

8. Utilisation d'un kit dans un procédé d'identification d'un état de cancer gastrique chez un sujet, comprenant une paire d'amorces pour détecter les niveaux de méthylation d'un gène biomarqueur dans un échantillon biologique provenant du sujet, dans laquelle la paire d'amorces est utilisée pour effectuer une réaction d'amplification par PCR avec le gène biomarqueur ou un fragment de celui-ci traité au bisulfite comme matrice; et les gènes biomarqueurs comprennent CDH1, DAPK, RASSF1A, RNF180 et Septin9,
dans lequel la paire d'amorces utilisée pour la détection du niveau de méthylation de CDH1 présente les séquences décrites dans les SEQ ID NOs : 11 et 12 ou les séquences décrites dans les SEQ ID NOs : 15 et 16 ;
la paire d'amorces utilisée pour la détection du niveau de méthylation de DAPK présente les séquences décrites dans les SEQ ID NOs : 19 et 20 ou les séquences décrites dans les SEQ ID NOs : 23 et 24;
la paire d'amorces utilisée pour la détection du niveau de méthylation de RASSF1A présente les séquences décrites dans les SEQ ID NOs : 35 et 36 ou les séquences décrites dans les SEQ ID NOs : 39 et 40 ;
la paire d'amorces utilisée pour la détection du niveau de méthylation de RNF180 présente les séquences décrites dans les SEQ ID NOs : 51 et 52, les séquences décrites dans les SEQ ID NOs : 55 et 56 ou les séquences décrites dans les SEQ ID NOs : 59 et 60; et
la paire d'amorces utilisée pour la détection du niveau de méthylation de Septin9 présente les séquences décrites dans les SEQ ID NOs : 87 et 88 ou les séquences décrites dans les SEQ ID NOs : 91 et 92.

9. Utilisation d'un kit dans un procédé d'identification d'un état de cancer gastrique selon la revendication 8, dans lequel les gènes de biomarqueurs sont CDH1, DAPK, PAX5, RASSF1A, Reprimo, RNF180, RUNX3, SDC2, Septin9 et TCF4.

10. Utilisation d'un kit dans un procédé d'identification d'un état de cancer gastrique selon la revendication 9, dans lequel
la paire d'amorces utilisée pour la détection du niveau de méthylation de CDH1 présente les séquences décrites dans les SEQ ID N° 11 et 12 ou les séquences décrites dans les SEQ ID N° 15 et 16;
la paire d'amorces utilisée pour la détection du niveau de méthylation de DAPK présente les séquences décrites dans les SEQ ID NOs : 19 et 20 ou les séquences décrites dans les SEQ ID NOs : 23 et 24;
la paire d'amorces utilisée pour la détection du niveau de méthylation de PAX5 présente les séquences décrites dans les SEQ ID NOs : 27 et 28 ou les séquences décrites dans les SEQ ID NOs : 31 et 32;
la paire d'amorces utilisée pour la détection du niveau de méthylation de RASSF1A présente les séquences décrites dans les SEQ ID NOs : 35 et 36 ou les séquences décrites dans les SEQ ID NOs : 39 et 40;
la paire d'amorces utilisée pour la détection du niveau de méthylation de Reprimo présente les séquences décrites dans les SEQ ID NOs : 43 et 44 ou les séquences décrites dans les SEQ ID NOs : 47 et 48;
la paire d'amorces utilisée pour la détection du niveau de méthylation de RNF180 présente les séquences décrites dans les SEQ ID NOs : 51 et 52, les séquences décrites dans les SEQ ID NOs : 55 et 56 ou les séquences décrites dans les SEQ ID NOs : 59 et 60;
la paire d'amorces utilisée pour la détection du niveau de méthylation de RUNX3 présente les séquences décrites dans les SEQ ID NOs : 63 et 64, les séquences décrites dans les SEQ ID NOs : 67 et 68, ou les séquences décrites dans les SEQ ID NOs : 71 et 72;
la paire d'amorces utilisée pour la détection du niveau de méthylation de SDC2 présente les séquences décrites dans les SEQ ID NOs : 75 et 76, les séquences décrites dans les SEQ ID NOs : 79 et 80 ou les séquences décrites dans les SEQ ID NOs : 83 et 84;
la paire d'amorces utilisée pour la détection du niveau de méthylation de Septin9 présente les séquences décrites dans les SEQ ID NOs : 87 et 88 ou les séquences décrites dans les SEQ ID NOs : 91 et 92; et
la paire d'amorces utilisée pour la détection du niveau de méthylation de TCF4 présente les séquences décrites dans les SEQ ID NOs : 95 et 96 ou les séquences décrites dans les SEQ ID NOs : 99 et 100.

11. Utilisation d'un kit dans un procédé d'identification d'un état de cancer gastrique de la revendication 10, en outre comprenant une amorce de blocage, dans lequel
l'amorce de blocage utilisée en combinaison avec la paire d'amorces ayant les séquences décrites dans les SEQ ID NO : 11 et 12 présente la séquence décrite dans la SEQ ID NO : 13;
l'amorce de blocage utilisée en combinaison avec la paire d'amorces ayant les séquences décrites dans les SEQ ID NO : 15 et 16 présente la séquence décrite dans la SEQ ID NO : 17;
l'amorce de blocage utilisée en association avec la paire d'amorces ayant les séquences décrites dans les SEQ ID NO : 19 et 20 présente la séquence décrite dans la SEQ ID NO : 21;
l'amorce de blocage utilisée en combinaison avec la paire d'amorces ayant les séquences décrites dans les SEQ ID NO : 23 et 24 présente la séquence décrite dans la SEQ ID NO : 25;
l'amorce de blocage utilisée en combinaison avec la paire d'amorces ayant les séquences décrites dans les SEQ ID NO : 27 et 28 présente la séquence décrite dans la SEQ ID NO : 29 ;
l'amorce de blocage utilisée en combinaison avec la paire d'amorces ayant les séquences décrites dans les SEQ ID NO : 31 et 32 présente la séquence décrite dans la SEQ ID NO : 33;
l'amorce de blocage utilisée en combinaison avec la paire d'amorces ayant les séquences décrites dans les SEQ ID NO : 35 et 36 présente la séquence décrite dans la SEQ ID NO : 37;
l'amorce de blocage utilisée en combinaison avec la paire d'amorces ayant les séquences décrites dans les SEQ ID NO : 39 et 40 présente la séquence décrite dans la SEQ ID NO : 41;
l'amorce de blocage utilisée en combinaison avec la paire d'amorces ayant les séquences décrites dans les SEQ ID NO : 43 et 44 présente a la séquence décrite dans la SEQ ID NO : 45;
l'amorce de blocage utilisée en combinaison avec la paire d'amorces ayant les séquences décrites dans les SEQ ID NO : 47 et 48 présente la séquence décrite dans la SEQ ID NO : 49;
l'amorce de blocage utilisée en combinaison avec la paire d'amorces ayant les séquences décrites dans les SEQ ID NO : 51 et 52 présente la séquence décrite dans la SEQ ID NO : 53;
l'amorce de blocage utilisée en combinaison avec la paire d'amorces ayant les séquences décrites dans les SEQ ID NO : 55 et 56 a la séquence décrite dans la SEQ ID NO : 57 ;
l'amorce de blocage utilisée en combinaison avec la paire d'amorces ayant les séquences décrites dans les SEQ ID NO : 59 et 60 présente la séquence décrite dans la SEQ ID NO : 61;
l'amorce de blocage utilisée en combinaison avec la paire d'amorces ayant les séquences décrites dans les SEQ ID NO : 63 et 64 présente la séquence décrite dans la SEQ ID NO : 65;
l'amorce de blocage utilisée en combinaison avec la paire d'amorces ayant les séquences décrites dans les SEQ ID NO : 67 et 68 présente a la séquence décrite dans la SEQ ID NO : 69
l'amorce de blocage utilisée en combinaison avec la paire d'amorces ayant les séquences décrites dans les SEQ ID NO : 71 et 72 présente la séquence décrite dans la SEQ ID NO : 73;
l'amorce de blocage utilisée en combinaison avec la paire d'amorces ayant les séquences décrites dans les SEQ ID NO : 75 et 76 présente la séquence décrite dans la SEQ ID NO : 77;
l'amorce de blocage utilisée en combinaison avec la paire d'amorces ayant les séquences décrites dans les SEQ ID NO : 79 et 80 présente la séquence décrite dans la SEQ ID NO : 81;
l'amorce de blocage utilisée en combinaison avec la paire d'amorces ayant les séquences décrites dans les SEQ ID NO : 83 et 84 présente la séquence décrite dans la SEQ ID NO : 85;
l'amorce de blocage utilisée en combinaison avec la paire d'amorces ayant les séquences décrites dans les SEQ ID NO : 87 et 88 présente la séquence décrite dans la SEQ ID NO : 89;
l'amorce de blocage utilisée en combinaison avec la paire d'amorces ayant les séquences décrites dans les SEQ ID NO : 91 et 92 a la séquence décrite dans la SEQ ID NO : 93 ;
l'amorce de blocage utilisée en combinaison avec la paire d'amorces ayant les séquences décrites dans les SEQ ID NO : 95 et 96 a la séquence décrite dans la SEQ ID NO : 97; et
l'amorce de blocage utilisée en combinaison avec la paire d'amorces ayant les séquences décrites dans les SEQ ID NO : 99 et 100 présente la séquence décrite dans la SEQ ID NO : 101,
dans lequel les amorces de blocage présentent une modification à l'extrémité 3' qui empêche l'extension et l'amplification par une ADN polymérase.

12. Utilisation d'un kit dans un procédé d'identification d'un état de cancer gastrique selon la revendication 8, comprenant en outre un réactif d'extraction d'ADN et un réactif au bisulfite.

13. Utilisation d'un kit dans un procédé d'identification d'un état de cancer gastrique selon la revendication 8, comprenant en outre une notice décrivant comment utiliser le kit et traiter les résultats de détection à l'aide d'une régression logistique.
